(19)

Europäisches
Patentamt
European
Patent Office
Office européen
des brevets

(11) **EP 4 249 498 A1**

(12) **EUROPEAN PATENT APPLICATION**

(43) Date of publication:
**27.09.2023 Bulletin 2023/39**

(21) Application number: **22382267.7**

(22) Date of filing: **23.03.2022**

(51) International Patent Classification (IPC):
**C07K 7/06** *(2006.01)*   **A23L 33/18** *(2016.01)*
**A61K 8/64** *(2006.01)*   **A61P 17/00** *(2006.01)*
**A61K 38/00** *(2006.01)*

(52) Cooperative Patent Classification (CPC):
**C07K 7/06; A61K 8/64; A61P 17/00;** A23L 33/18;
A61K 38/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Lipotrue, S.L.**
**08850 Gava (ES)**

(72) Inventors:
 • **Grau-Campistany, Ariadna**
  **08028 Barcelona (ES)**
 • **Pastor, Silvia**
  **03540 Alicante (ES)**

 • **Carulla, Patricia**
  **08019 Barcelona (ES)**
 • **Escudero, Juan Carlos**
  **08021 Barcelona (ES)**
 • **Klein, Marco Jan**
  **08902 L'Hospitalet de Llobregat (ES)**

(74) Representative: **Clarke, Modet y Cía., S.L.**
 **C/ Suero de Quiñones 34-36**
 **28002 Madrid (ES)**

Remarks:
The complete document including Reference
Table(s) and the Sequence Listing(s) can be
downloaded from the EPO website

(54) **PEPTIDES AND COMPOSITIONS FOR USE IN COSMETICS, FOOD AND MEDICINE**

(57) The present invention refers to peptides which provide for the protection of telomeres, antioxidant and anti-inflammatory activities and, hence, are useful in cosmetics for the prevention, reduction and/or treatment of inflammaging and skin aging as well as in medicine for the treatment of age-related diseases, inflammatory diseases and diseases related to oxidative-stress; and in the food industry as nutraceuticals.

EP 4 249 498 A1

Fig. 1

**Description**

The present invention relates to the field of molecular biology, more precisely to molecular biology applied to cosmetics, nutraceuticals and medicine, even more precisely to peptides and compositions comprising said peptides, able, among others, to protect telomers and to protect from inflammation and oxidation, therefore, said peptides provide for the prevention and/or treatment of aging (preferably, skin aging), including inflammaging.

In the last decades life expectancy of the population has increased significantly. In addition, there is also an increased concern in the population regarding personal aesthetics and to try to delay or minimize the appearance of signs related to aging.

The skin is the largest organ in humans and due to its location, in the body interface, is subject to intrinsic (chronologic) aging and extrinsic aging (mainly, photoaging). Intrinsic aging is accompanied by cell loss, thinning of epidermis, flattening of DEJ (dermal-epidermal junction) and fine lines of wrinkles. On the other hand, photoaged skin is coarsely wrinkled and associated with dyspigmentation. Inflammation and accumulation of ROS (reactive oxygen species) are now believed to be the causative factors in both types of skin aging.

One of the main functions of the skin is to protect the body from external factors and it does so by employing physical barriers, biomolecules, and an intricate network of resident immune and non-immune cells and skin structures. Concretely, the most external layer of the skin, named the epidermis, provides a barrier that confers the protection from environmental factors, pathogens, heat, ultraviolet and moisture loss. In addition, keratinocytes are keratin-producing epidermal cells and account for approximately 90% of epidermal cells (Nguyen AV, & Soulika AM (2019) The Dynamics of the Skin's Immune System. International journal of molecular sciences, 20(8), 1811; Lee HS, et al. (2019) Anti-inflammatory effects of ethanol extract from the leaves and shoots of Cedrela odorata L. in cytokine-stimulated keratinocytes. Experimental and therapeutic medicine, 18(1), 833-840).

Skin aging is a complex biological process influenced by combination of endogenous and exogenous factors (endogenous: genetics, cellular metabolism, hormone and metabolic processes; exogenous: chronic light exposure, pollution, ionizing radiation, chemicals, toxins) and, hence, as noted above, lead to changes in the skin and to the appearance of imperfections therein (for example, loss of firmness, wrinkling, roughness and/or sagginess).

These factors induce cumulative structural and physiological alterations at the different layers of the skin as well as changes in overall skin appearance (Ganceviciene, R.,, et al. (2012) Skin Anti-Aging Strategies. Dermato-Endocrinology, 4, 308-319).

As the aging process occurs, the ability of the human body to resolve inflammation becomes significantly reduced, resulting in an imbalance between proinflammation and anti-inflammation. "Inflammaging" refers to a continuous, low-grade inflammation associated with aging. Such chronic inflammatory response could build up with time and gradually causes tissue damage (Zhuang Y, Lyga J (2014) Inflammaging in skin and other tissues - the roles of complement system and macrophage. Inflamm Allergy Drug Targets. 13, 153-61). Inflammaging is driven by a systemic increase in multiple proinflammatory cytokines and is significantly influenced by several extrinsic factors, including UV radiation (UVR), air particulate matter (PM), and the microbiome (Xia S, et al. (2016) An Update on Inflamm-Aging: Mechanisms, Prevention, and Treatment. J Immunol Res. 2016, 8426874).

In addition, or in parallel, UV irradiation, xenobiotics, and thermal stress disturb skin cell metabolism and lead to an increase in reactive oxygen species (ROS) generation and to redox imbalance contributing to oxidative stress which exposes skin cells to the formation and accumulation of irreversibly damaged proteins, lipids, nucleic acids and carbohydrates and, hence to skin aging. Key elements or mechanisms of skin cells against oxidative stress are Nrf2 (able to modulate anti-inflammatory and antioxidant response through multiple mechanisms, such as the regulation of redox homeostasis and the suppression of pro-inflammatory genes, either directly or through the interaction with NF-κB) and catalase (crucial antioxidant enzymes that mitigates oxidative stress to a considerable extent by destroying cellular hydrogen peroxide to produce water and oxygen).

Senescence typically occurs in response to damaging stimuli, such as telomere shortening or dysfunction (replicative senescence) or persistent activation of the DNA-damage response (DDR). The adoption of a pro-inflammatory phenotype known as the senescence-associated secretory phenotype (SASP) may promote tissue inflammation and deterioration. Growing evidence suggests that senescent cells accumulate in tissues and organs with advancing age, thereby impairing physiological regeneration processes and contributing to organismal aging (Rübe, C.E., et al. (2021) Human skin aging is associated with increased expression of the histone variant H2A.J in the epidermis. Aging Mech Dis 7, 7). Moreover, as noted above, ROS production increases with age, causing also double strand breaks (DSBs) in DNA while skin cells ability to repair this damage decreases over the years, contributing to chronological aging. It has been shown that during RS (replicative senescence), fibroblasts accumulate proteins involved in the DDR at telomeric regions, including γH2A.X, 53BP1, MDC1 and NBS1 (d'Adda di Fagagna et al., (2003) A DNA damage checkpoint response in telomere-initiated senescence, Nature 426, 194-198). DDR activation can result in the activation of transcription factor p53 which is involved in a variety of processes including DNA repair and apoptosis, but most notably results in expression of cyclin-dependent kinase inhibitor p21, which together with activation of p16, are thought to be the major pathways in the induction of senescence (de Magalhães J P, Passos J F, (2018) Stress, cell senescence and organismal ageing; Mechanisms of Ageing and Development, Volume 170, 2-9). Antioxidant substances have the ability to bind free radicals, which are caused by oxidative stress, reducing DNA DSBs, and therefore, the apparition of 53BP1 foci, preventing and slowing the skin aging process.

On its side, Interleukin-8 (IL-8) is a potent chemotactic and proinflammatory cytokine, produced in the skin by a variety of cells in response to inflammatory stimuli. Recent studies suggest that in addition to its potent actions on leukocytes, IL-8 exerts a direct influence on several functions of human epidermal cells such as chemotaxis, *Candida albicans* killing activity or proliferation (Kemény L, et al. (1994) Role of interleukin-8 receptor in skin. International archives of allergy and immunology, 104(4), 317-322.).

In addition, one of the main players in innate immune response is toll-like receptor 2 (TLR2). This protein is a membrane receptor involved in recognizing pathogen-associated molecular patterns (PAMPs) or exogenous ligands, and also recognizes danger associated molecular patterns (DAMPs), also known as alarmins or endogenous ligands. This recognition leads to the activation of the proinflammatory response in order to protect the organisms against external insults (Chen L, DiPietro LA (2017) Toll-Like Receptor Function in Acute Wounds. Adv Wound Care (New Rochelle). 6, 344-355). Focusing on the skin, which main function is to protect the body from external factors, TLR2 is expressed in many of the cell types that compose this organ, including keratinocytes (Nguyen AV, & Soulika AM (2019) The Dynamics of the Skin's Immune System. International journal of molecular sciences, 20(8), 1811; Pivarcsi A, et al. (2003) Expression and function of Toll-like receptors 2 and 4 in human keratinocytes. Int Immunol. 15, 721-30). Misfunction or overactivation of TLR2 in skin, can lead to an excessive pro-inflammatory response, causing inflammation-related diseases like acne and contributing to maintain a sustained inflammatory state, that can lead to inflammaging (Bailey KL, et al.(2019) Aging leads to dysfunctional innate immune responses to TLR2 and TLR4 agonists. Aging Clin Exp Res. 31, 1185-1193.; Zhang B, et al. (2019) Toll-like receptor 2 plays a critical role in pathogenesis of acne vulgaris. Biomed Dermatol 3, 4).

Moreover, it is important to highlight that telomeres form the ends of human chromosomes. These structures consist of simple tandem DNA repeats (TTAGGG), that do not encode for any gene product and their main function is to cap the chromosome ends. Telomere capping is necessary to distinguish the chromosome ends, that should not be repaired, from DNA breaks within the genome, which lead to cell cycle arrest and DNA repair or to induction of apoptosis when the damage is too severe or non-repairable. Telomeres prevent the induction of such responses at the chromosome termini and this capping is a prerequisite to maintain chromosome stability, being necessary a minimum telomere length to fulfil this capping function (Buckingham EM, Klingelhutz AJ (2011) The role of telomeres in the ageing of human skin. Exp Dermatol. 20, 297-302; Jiang H, et al. (2007) Telomere shortening and ageing. Z Gerontol Geriatr. 40, 314-24).

Telomers shorten with each round of cell division, due to the "end replication problem" of DNA-polymerase, being one of the most relevant features of aging. Additional factors (processing of telomeres during the cell cycle, reactive oxygen species) can contribute to telomere shortening and different cellular mechanisms are involved in telomere preservation. Among these mechanisms, the sheltering complex is essential for telomere function, telomere maintenance, and connections with intracellular signalling pathways. Said sheltering complex is a multi-protein structure that protects telomere endings and it is formed by different proteins, including TRF1, TRF2, POT1, RAP1, TIN2, and TPP1 (Imbert I, et al. (2012) Modulation of telomere binding proteins: a future area of research for skin protection and anti-aging target. J Cosmet Dermatol. 11, 162-6).

When telomeres reach a critically short length, they lose the capping function at the chromosome termini. These dysfunctional telomeres are then recognized as DNA damage and induce DNA damage checkpoints, leading to senescence or even cell death (Imbert I, et al. (2012) Modulation of telomere binding proteins: a future area of research for skin protection and anti-aging target. J Cosmet Dermatol. 11, 162-6; Maleki M, et al. (2020) Stabilization of telomere by the antioxidant property of polyphenols: Anti-aging potential. Life Sci. 259, 118341.). Focusing in skin aging, accumulation of senescent cells with short telomeres in skin tissues, is a hallmark of the ageing process (Buckingham EM, Klingelhutz AJ (2011) The role of telomeres in the ageing of human skin. Exp Dermatol. 20, 297-302; Jiang H, et al. (2007) Telomere shortening and ageing. Z Gerontol Geriatr. 40, 314-24).

Telomerase is an enzyme that elongates telomeres and compensates for telomere attrition through de novo addition of TTAGGG repeats onto chromosome ends in those cells where it is normally expressed, such as pluripotent stem cells and adult stem cell compartments (Blackbum, E. H. (1991) Structure and function of telomeres. Nature 350, 569-573.; Liu, L., et al. (2007) Telomere lengthening early in development. Nat. Cell Biol. 9:1436- 1441; Flores, I., et al. (2005) Effects of telomerase and telomere length on epidermal stem cell behavior. Science. 309: 1253-1256; Marion, R.M., et al. (2009) Telomeres acquire embryonic stem cell characteristics in induced pluripotent stem cells. Cell Stem Cell. 4:141-54). Although telomerase is expressed in adult stem cell compartments, this is not sufficient to counteract telomere attrition associated with cell division throughout life, and therefore, as noted above, telomeres shorten with age in vitro and in vivo. This progressive telomere shortening can impair the regenerative capacity of tissues and has been proposed as one of the molecular hallmarks of aging (López-Otín, C., et al. (2013) The hallmarks of aging. Cell.153:1194-1217.; Martinez, P, Blasco, M.A. (2017) Telomere-driven diseases and telomere-targeting therapies. J Cell Biol. 216(4):875-887.). Telomere attrition in stem cell compartments impairs their tissue and self-renewal capacity and is considered to be one of the primary molecular causes of aging and the onset of aging-associated diseases (Flores, I., et al. (2005) Effects of telomerase and telomere length on epidermal stem cell behavior. Science. 309: 1253-1256.: Sharpless., N.E. and DePinho, R.A. (2007) How stem cells age and why this makes us grow old. Nat. Rev. Mol. Cell Biol. 8:703-713). Specifically, for skin aging, telomerase, has been implicated in having a key role in the maintenance of skin cell function and proliferation (Buckingham EM, Klingelhutz AJ (2011) The role of telomeres in the ageing of human skin. Exp Dermatol. 20, 297-302).

In recent years, the number of active ingredients to improve signs of skin aging, such as loss of firmness, skin texture and wrinkling, has increased considerably. Examples of such active ingredients are retinoids, vitamins or botanical extracts (Bradley E.J., et al. (2015), Over-the-counter anti-ageing topical agents and their ability to protect and repair photoaged skin. Maturitas, 80, 265-272).

Antioxidants are used in order to reduce the concentration of free radicals in the skin and, therefore, counteract collagen degradation. An example of said antioxidants is ascorbic acid (also known as Vitamin C). Ascorbic acid, in addition to its antioxidant effect, induces the synthesis of proteins from the ECM (collagen I and III and elastin) while promoting epidermal differentiation and inhibiting Matrix metalloproteinase-1 (MMP1), among others. Unfortunately, ascorbic acid, is extremely unstable and undergoes oxidation especially at high temperatures, aerobic conditions, high pH and/or when exposed to light (Manela-Azulay M., et al. (2017), Vitamins and other Antioxidants. Daily Routine in Cosmetic Dermatology, 1-13).

In addition, a wide range of botanical extracts and plant derived compounds are found in the market with multiple applications, such as, for example, grape extracts which comprise resveratrol (an antioxidant); green tea which comprises polyphenols; or soy which comprises isoflavones. Another example is turmeric (*Curcuma longa)*, which was used in Chinese and Ayurvedic medicine (as a treatment for inflammatory conditions) and heralded for its use in cooking and beauty rituals around the world. Turmeric comprises turmerin, which has shown antioxidant and anti-inflammatory activities.

However, the in vivo efficacy and composition of these ingredients is not sufficiently scientifically validated.

On the other hand, peptides can also be incorporated in cosmetic formulas to improve the signs of skin aging. Bioactive peptides can imitate body's own molecules and influence processes such as collagen synthesis, with the advantage that they have much better tolerability and stability. In addition, a wide range of activities, chemistries and indications can be developed for them (Zhang L. and Falla T.J. (2009), Cosmeceuticals and peptides. Clinics in dermatology, 27, 485-494).

Despite the extensive variety of compounds and/or extracts in the field, there is still the need to find new molecules (preferably, peptides) which can provide for the prevention and/or treatment of aging (preferably, skin aging) and which show a broad spectrum of activities against said aging as well as diseases related to aging, inflammation and oxidative stress.

The inventors of the present invention, after extensive and exhaustive research, have surprisingly found peptides with a broad spectrum of activities which, among others, include protection of telomeres and antioxidative and anti-inflammatory activities. These peptides of the present invention are, therefore, useful for the treatment of skin aging and signs related to said skin aging as well as inflammatory diseases and diseases related with oxidative stress and, hence, solve the problem present in the state of the art mentioned above.

In a first aspect, the present invention refers to a peptide with a broad spectrum of activities against skin aging which include, protection of telomeres and anti-inflammatory and antioxidant activities.

In a second aspect, the present invention refers to a composition comprising a peptide of the present invention.

In an additional aspect, the present invention refers to the use as a cosmetic of a peptide of the present invention or of a composition of the present invention, in a subject in need of the treatment.

In a fourth aspect, the present invention refers to the cosmetic use of a peptide of the present invention or a composition of the present invention in a subject in need of the treatment.

In a fifth aspect, the present invention refers to a method for the prevention and/or treatment of skin aging in a subject comprising the administration of a peptide of the present invention or a composition of the present invention to the subject.

In a further aspect, the present invention refers to a composition or a peptide of the present invention for use as a medicament.

In a seventh aspect, the present invention refers to the use of the composition or a peptide of the present invention for the manufacture of a medicament for the prevention, amelioration and/or treatment of an age-related disease, an inflammatory disease or a disease related to oxidative stress.

In an eighth aspect, the present invention refers to a method for the prevention, amelioration and/or treatment of an age-related disease, an inflammatory disease or a disease related to oxidative stress, comprising administering a peptide or a composition of the present invention to a subject in need thereof.

In a ninth aspect, the present invention refers to a nutraceutical composition comprising a peptide of the present invention.

In a final aspect, the present invention refers to the use as a nutraceutical of a peptide of the present invention or of a composition of the present invention.

The term "non-cyclic aliphatic group" and its plural, as used herein, have the common meaning given in the state of the art to said terms. Therefore, these terms refer to, for example and not restricted to, linear or branched alkyl, alkenyl and alkynyl groups.

The term "alkyl group" and its plural, as used herein, refer to a saturated, linear or branched group, which has between 1 and 24, preferably between 1 and 16, more preferably between 1 and 14, even more preferably between 1 and 12, and even more preferably still between 1, 2, 3, 4, 5 or 6 carbon atoms and which is bound to the rest of the molecule by a simple bond, including, for example and not restricted to, methyl, ethyl, isopropyl, n-propyl, i-propyl, isobutyl, tert-butyl, n-butyl, sec-butyl, n-pentyl, n-hexyl, heptyl, octyl, decyl, dodecyl, lauryl, hexadecyl, octadecyl, amyl, 2-ethylhexyl, 2-methylbutyl, 5-methylhexyl and similar. The alkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alkenyl group" and its plural, as used herein, refer to a linear or branched group which has between 2 and 24, preferably between 2 and 16, more preferably between 2 and 14, even more preferably between 2 and 12, even more preferably still 2, 3, 4, 5 or 6 carbon atoms, with one or more carbon-carbon double bonds, preferably with 1, 2 or 3 carbon-carbon double bonds, conjugated or unconjugated, which is bound to the rest of the molecule through a single bond, including, for example and not restricted to, the vinyl, oleyl, linoleyl and similar groups. The alkenyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alkynyl group" and its plural, as used herein, refer to a linear or branched group which has between 2 and 24, preferably between 2 and 16, more preferably between 2 and 14, even more preferably between 2 and 12, even more preferably still 2, 3, 4, 5 or 6 carbon atoms, with one or more carbon-carbon triple bonds, preferably with 1, 2 or 3 carbon-carbon triple bonds, conjugated or unconjugated, which is bound to the rest of the molecule through a single bond, including, for example and not restricted to, the ethinyl group, 1-propinyl, 2-propinyl, 1-butinyl, 2-butinyl, 3-butinyl, pentinyl, such as 1-pentinyl and similar groups. The alkynyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alicyclic group" and its plural, as used herein, have the common meaning given in the state of the art to said terms. Hence, these terms are used to refer to, for example and not restricted to, cycloalkyl or cycloalkenyl or cycloalkynyl groups.

The term "cycloalkyl" and its plural, as used herein, refer to a saturated mono- or polycyclic aliphatic group which has between 3 and 24, preferably between 3 and 16, more preferably between 3 and 14, even more preferably between 3 and 12, even more preferably still 3, 4, 5 or 6 carbon atoms and which is bound to the rest of the molecule through a single bond, including, for example and not limited to, cyclopropyl, cyclobutyl, cyclopentyl, cyclohexyl, cycloheptyl, methyl cyclohexyl, dimethyl cyclohexyl, octahydroindene, decahydronaphthalene, dodecahydro-phenalene, adamantyl and similar, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "cycloalkenyl" and its plural, as used herein, refer to a non-aromatic mono- or polycyclic aliphatic group which has between 5 and 24, preferably between 5 and 16, more preferably between 5 and 14, even more preferably between 5 and 12, even more preferably still 5 or 6 carbon atoms, with one or more carbon-carbon double bonds, preferably with 1, 2 or 3 carbon-carbon double bonds, conjugated or unconjugated, which is bound to the rest of the molecule through a single bond, including, for example and not restricted to, the cyclopent-1-en-1-yl group and similar groups, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "cycloalkynyl" and its plural, as used herein, refer to a non-aromatic mono- or polycyclic aliphatic group which has between 8 and 24, preferably between 8 and 16, more preferably between 8 and 14, even more preferably between 8 and 12, even more preferably still 8 or 9 carbon atoms, with one or more carbon-carbon triple bonds, preferably with 1, 2 or 3 carbon-carbon triple bonds, conjugated or unconjugated, which is bound to the rest of the molecule through a single bond, including, for example and not restricted to, the cyclooct-2-yn-1-yl group and similar, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "aryl group" and its plural, as used herein, refer to an aromatic group which has between 6 and 30, preferably between 6 and 18, more preferably between 6 and 10, even more preferably 6 or 10 carbon atoms, which comprises 1, 2, 3 or 4 aromatic rings, bound by a carbon-carbon bond or fused, and which is bound to the rest of the molecule through a single bond, including, for example and not restricted to, phenyl, naphthyl, diphenyl, indenyl, phenanthryl or anthranyl among others. The aryl group can be optionally substituted by one or more substituents, such as, halo, hydroxy, alkoxy, carboxy, carbonyl, cyano, acyl, alkoxy-carbonyl, amino, nitro, mercapto and alkoxythio.

The term "aralkyl group" and its plural, as used herein, refer to an alkyl group substituted by an aromatic group, with between 7 and 24 carbon atoms and including, for example and not restricted to, -$(CH_2)$1-6-phenyl, -$(CH_2)$1-6-(1-naphtyl), -$(CH_2)$1-6-(2-naphtyl), - $(CH_2)$1-6-CH(phenyl)$_2$ and similar. The aralkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "heterocyclic group" and its plural, as used herein, refer to a 3-10 member heterocycyl or hydrocarbon ring, in which one or more of the ring atoms, preferably 1, 2 or 3 of the ring atoms, is a different element to carbon, such as nitrogen, oxygen or sulfur and may be saturated or unsaturated. For the purposes of this invention, the heterocyclyl can be a cyclic, monocyclic, bicyclic or tricyclic system which may include fused ring systems; and the nitrogen, carbon or sulfur atoms can be optionally oxidized in the heterocyclyl radical; the nitrogen atom can optionally be quaternized; and the heterocyclyl radical may be partially or completely saturated or may be aromatic. With increasing preference, the term heterocyclic relates to a 5 or 6-member ring. The heterocyclic groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "heteroarylalkyl group" and its plural, as used herein, refer to an alkyl group substituted with a substituted or unsubstituted aromatic heterocyclyl group, the alkyl group having from 1 to 6 carbon atoms and the aromatic heterocyclyl group between 2 and 24 carbon atoms and from 1 to 3 atoms other than carbon and including, for example and not restricted to, -$(CH_2)$1-6-imidazolyl, -$(CH_2)$1-6-triazolyl, -$(CH_2)$1-6-thienyl, - $(CH_2)$1-6-furyl, -$(CH_2)$1-6-pyrrolidinyl and similar. The heteroarylalkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The terms "halo" or "halogen", as used in the present document, refer to fluorine, chlorine, bromine or iodine, and its anions are referred to as halides.

As used herein, the term "derivative" and its plural, refer to cosmetically, pharmaceutically and/or food acceptable compounds, this is, derived from the compound of interest that can be used in the preparation of a cosmetic, pharmaceutical and/or food composition, and to cosmetically, pharmaceutically and/or food unacceptable derivatives since these may be useful in the preparation of cosmetically, pharmaceutically and/or food acceptable derivatives.

As used in the present document, the term "salt" and its plurals refer to any type of salt from among those known in the state of the art, for example, halide salts, hydroxy acid salts (such as oxyacid salts, acid salts, basic salts and double salts), hydroxo salts, mixed salts, oxy salts or other hydrated salts. This term comprises cosmetically, pharmaceutically and/or food acceptable salts; and cosmetically, pharmaceutically and/or food unacceptable salts, since the latter may be useful in the preparation of cosmetically, pharmaceutically and/or food acceptable salts.

As used in the present document, the term "isomer" and its plural refer to optical isomers, enantiomers, stereoisomers or diastereoisomers. The individual enantiomers or diastereoisomers, as well as their mixtures, may be separated by conventional techniques known in the state of the art.

As used herein, the term "solvate" and its plural refer to any solvate known in the state of the art, such as polar, apolar or amphiphilic solvates, and include any cosmetically, pharmaceutically and/or food acceptable solvate which, when administered or applied to the interested subject (directly or indirectly) provides the compound of interest (the peptide or peptides of the present invention). Preferably, the solvate is a hydrate, a solvate with an alcohol such as methanol, ethanol, propanol or isopropanol, a solvate with an ester such as ethyl acetate, a solvate with an ether such as methyl ether, ethyl ether or THF (tetrahydrofuran) or a solvate with DMF (dimethylformamide), and more preferably a hydrate or a solvate with an alcohol such as ethanol.

In addition, as used herein, the term "amino acid" and its plural include the amino acids codified by the genetic code as well as uncodified amino acids, whether they are natural or not and whether they are D- and L-amino acids. Examples of uncodified amino acids are, without restriction, citrulline, ornithine, sarcosine, desmosine, norvaline, 4-aminobutyric acid, 2-aminobutyric acid, 2-aminoisobutyric acid, 6-aminohexanoic acid, 1-naphthylalanine, 2-naphthylalanine, 2-aminobenzoic acid, 4 aminobenzoic acid, 4-chlorophenylalanine, 2,3-diaminopropionic acid, 2,4 diaminobutyric acid, cycloserine, carnitine, cysteine, penicillamine, pyroglutamic acid, thienylalanine, hydroxyproline, alloisoleucine, allo-threonine, isonipecotic acid, isoserine, phenylglycine, statin, β-alanine, norleucine, N-methylamino acids, α-amino acids and β-amino acids, among others, as well as their derivatives. Nevertheless, further unnatural amino acids are known in the state of the art (see, for example, "Unusual amino acids in peptide synthesis" by D. C. Roberts and F. Vellaccio, The Peptides, Vol. 5 (1983), Chapter VI, Gross E. and Meienhofer J., Eds., Academic Press, New York, USA). The abbreviations used in the present text for amino acids follow the 1983 IUPAC-IUB Joint Commission on Biochemical Nomenclature recommendations outlined in Eur. J. Biochem. (1984) 138:937.

The "percentage of identity" regarding peptides, polypeptides and proteins, as used herein, has the meaning commonly attributed in the state of the art and, hence, relates to the percentage of amino acids which are identical between two amino acid sequences which are compared after an optimal alignment of these sequences, where said percentage is merely statistical and the differences between the two amino acid sequences are randomly distributed throughout the sequence. "Optimal alignment" is understood as that alignment of amino acid sequences giving rise to a greater percentage of identity. The percentage of identity is calculated by determining the number of identical positions in which an amino acid is identical in the two compared sequences, dividing the number of identical positions by the number of compared positions and multiplying the result obtained by 100 to obtain the percentage of identity between the two sequences. The sequence comparisons between two amino acid sequences can be carried out manually or by means of computer programs known in the state of the art, such as the BLAST (Basic Local Alignment Search Tool) algorithm.

The terms "inflammaging" and "inflamm-aging" are used herein interchangeably and acquire the meaning they commonly have in the state of the art, this is, they refer to the chronic, low-grade inflammation that characterizes aging.

Therefore, in a first aspect, the present invention refers to a peptide of formula (I):

$$R_1\text{-AA1-AA2-AA3-AA4-AA5-AA6-AA7-AA8-}R_2 \qquad \text{(I)}$$

($R_1$-SEQ ID NO: 1-$R_2$)
its acceptable isomers, salts, solvates, derivatives and/or mixtures thereof, wherein

AA1 is a small amino acid;
AA2 is selected from polar uncharged or aromatic amino acids;
AA3 is absent or selected from aromatic amino acids;
AA4 is selected from negatively charged amino acids;
AA5 is selected from apolar amino acids;
AA6 is selected from Asn or Leu;
AA7 is selected from negatively charged amino acid;
AA8 is absent or selected from aromatic amino acids;
$R_1$ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and $R_5$-CO-, wherein $R_5$ is selected from the group formed by substituted or unsubstituted $C_1$-$C_{24}$ alkyl radical, substituted or unsubstituted $C_2$-$C_{24}$ alkenyl, substituted or unsubstituted $C_2$-$C_{24}$ alkynyl, substituted or unsubstituted $C_3$-$C_{24}$ cycloalkyl, substituted or unsubstituted $C_5$-$C_{24}$ cycloalkenyl, substituted or unsubstituted $C_8$-$C_{24}$ cycloalkynyl, substituted or unsubstituted $C_6$-$C_{30}$ aryl, substituted or unsubstituted $C_7$-$C_{24}$ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and $R_2$ is selected from H, -$NR_3R_4$- , -$OR_3$ and -$SR_3$, wherein $R_3$ and $R_4$ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl;
alternatively, $R_1$ and $R_2$ are absent and AA1 is directly bound to the last amino acid of the peptide, this is, AA8 or AA7 in case AA8 is absent (forming a cyclic peptide).

$R_1$ is linked to the N-terminal of the peptide, this is, to the amino group of AA1 (it substitutes or replaces an H from the amino group of AA1).

$R_2$ is linked to the C-terminal of the peptide, this is, to the carboxy group of AA8 or in case AA8 is absent to the carboxy group of AA7 (it substitutes or replaces the OH of the carboxy group).

It is contemplated that the amino acids used or present in the peptides of the present invention are L-amino acids, D-amino acids or combinations thereof. In a preferred embodiment, the amino acids used or present in the peptides of the present invention are L-amino acids.

Preferably, the isomers mentioned above are stereoisomers. It is contemplated that said stereoisomers are enantiomers or diastereoisomers. Hence, in a preferred embodiment of the present invention, the peptide is a racemic mixture, a diastereomeric mixture, a pure enantiomer or a pure diastereoisomer.

In addition, isomers, salts, solvates, derivatives and/or mixtures thereof are, preferably, cosmetically, pharmaceutically and/or food acceptable isomers, salts, solvates, derivatives and/or mixtures thereof.

Preferably, AA1 is selected from Cys, Val, Ala, Pro, Ser, Asn, Ala, Gly or Thr, more preferably, AA1 is selected from Ser, Cys, Ala or Gly; even more preferably, AA1 is Gly.

Also preferably, AA2 is selected from Tyr, Phe, Trp, Ser, Thr, Gln, Asn, Cys or Gly, more preferably AA2 is selected from Tyr, Trp, Ser, Asn or Gln, more preferably AA2 is selected from Tyr or Asn.

Preferably, AA3 is absent or selected from Tyr, Phe or Trp, more preferably AA3 is absent or Tyr.

In the present invention, AA4 is, preferably, selected from Asp or Glu, more preferably AA4 is Glu.

Also preferably, AA5 is selected from Ala, Val, Ile, Leu, Met, Pro, Phe or Trp, more preferably AA5 is selected from Ala, Ile, Leu or Val, even more preferably AA5 is Leu.

In the present invention, AA7 is, preferably, selected from Asp or Glu, more preferably AA7 is Asp.

Preferably, AA8 is absent or selected from Tyr, Phe or Trp, more preferably AA8 is absent or Tyr.

Therefore, in a preferred embodiment, in formula (I):

AA1 is selected from Cys, Val, Ala, Pro, Ser, Asn, Ala, Gly or Thr;
AA2 is selected from Tyr, Ser, Thr, Gln, Asn, Cys or Gly;
AA3 is absent or selected from Tyr, Phe or Trp;
AA4 is selected from Asp or Glu;
AA5 is selected from Ala, Val, Ile, Leu, Met, Pro, Phe or Trp;
AA6 is selected from Asn or Leu;
AA7 is selected from Asp or Glu;
AA8 is absent or selected from Tyr, Phe or Trp;
more preferably, in formula (I):

AA1 is selected from Ser, Cys, Ala or Gly;
AA2 is selected from Tyr, Trp, Ser, Gln or Asn (more preferably, Tyr or Asn);
AA3 is absent or selected from Tyr, Phe or Trp (more preferably, absent or Tyr);
AA4 is selected from Asp or Glu (more preferably, Glu);
AA5 is selected from Ala, Val, Ile or Leu (more preferably, Leu);
AA6 is selected from Asn or Leu;
AA7 is selected from Asp or Glu (more preferably, Asp);
AA8 is absent or selected from Tyr, Phe or Trp (more preferably, absent or Tyr);

even more preferably, in formula (I):

AA1 is Gly;
AA2 is selected from Tyr or Asn;
AA3 is absent or Tyr;
AA4 is Glu;
AA5 is Leu;
AA6 is selected from Asn or Leu;
AA7 is Asp;
AA8 is absent or Tyr.

In any of the above embodiments, in case AA3 is absent, preferably AA8 is not absent and vice versa. This is, in a preferred embodiment, only one of AA3 and AA8 is absent.

Therefore, in an embodiment, AA3 and AA8 are both present. In another embodiment, AA3 and AA8 are both absent. However, in a most preferred embodiment, AA3 is absent and AA8 is present; and in another most preferred embodiment AA3 is present and AA8 absent.

In a preferred embodiment, the peptide of the present invention is:

- $R_1$-Gly-Tyr-Tyr-Glu-Leu-Asn-Asp-$R_2$ ($R_1$-SEQ ID NO: 2-$R_2$); or
- $R_1$-Gly-Asn-Glu-Leu-Leu-Asp-Tyr-$R_2$ ($R_1$-SEQ ID NO: 3-$R_2$)

being $R_1$ and $R_2$ as disclosed above.

More preferably, the peptide of the present invention is:

- $R_1$-L-Gly-L-Tyr-L-Tyr-L-Glu-L-Leu-L-Asn-L-Asp-$R_2$ ($R_1$-SEQ ID NO: 2-$R_2$); or
- $R_1$-L-Gly-L-Asn-L-Glu-L-Leu-L-Leu-L-Asp-L-Tyr-$R_2$ ($R_1$-SEQ ID NO: 3-$R_2$)

In any of the above embodiments, preferably, $R_1$ is H or acetyl, more preferably $R_1$ is H.

Also, in any of the above embodiments, $R_2$ is $NH_2$ or OH, more preferably $R_2$ is $NH_2$.

Hence, in a most preferred embodiment, $R_1$ is H and $R_2$ is $NH_2$.

In the most preferred embodiment, the peptide of the present invention is:

- Gly-Tyr-Tyr-Glu-Leu-Asn-Asp-$NH_2$ (SEQ ID NO: 2-$NH_2$); or
- Gly-Asn-Glu-Leu-Leu-Asp-Tyr-$NH_2$ (SEQ ID NO: 3-$NH_2$)

even more preferably, the peptide of the present invention is:

- L-Gly-L-Tyr-L-Tyr-L-Glu-L-Leu-L-Asn-L-Asp-NH$_2$ (SEQ ID NO: 2-NH$_2$); or
- L-Gly-L-Asn-L-Glu-L-Leu-L-Leu-L-Asp-L-Tyr-NH$_2$ (SEQ ID NO: 3-NH$_2$)

Peptide SEQ ID NO: 2 is a biomimetic peptide derived or generated from Turmerin protein (*Curcuma longa*) - UniProt ref P85278 (TURM_CURLO).

Peptide SEQ ID NO: 3 is a biomimetic peptide derived or generated from Ricin protein (*Ricinus communis* (Castor bean)) - UniProt ref P02879 (RICI_RICCO).

In a preferred embodiment, combinable with any of the above embodiments, the peptide of the present invention provides for a protection of the telomeres, preferably by increasing the expression and/or levels of TRF1.

In a further preferred embodiment, combinable with any of the above embodiments, the peptide of the present invention provides for an increase or a recovery in the length in telomeres.

In a further preferred embodiment, combinable with any of the above embodiments, the peptide of the present invention provides for antioxidant activity, preferably by means of: increasing the levels of catalase, translocation of Nfr2 into the nuclei of cells or combinations thereof, more preferably, by means of increasing the levels of catalase and translocation of Nfr2 into the nuclei of cells.

In a further preferred embodiment, combinable with any of the above embodiments, the peptide of the present invention provides for anti-inflammatory activity, preferably by means of modulation of inflammation-related genes and/or miRNAs, decreasing the levels of IL-8 and/or reducing the levels of TLR2 or combinations thereof, most preferably at least by decreasing the levels of IL-8.

Modulation of inflammation-related genes is preferably upregulation of genes TNFAIP3, TOLLP, HMOX1, IRAK3, NQO1 and downregulation of genes NFKB1, IL1B, TNF, CXCL8, IL6, MMP3 and TLR2.

Modulation of inflammation-related miRNAs is preferably upregulation of miR-146a-5p and miR-21-5p.

Within the scope of the present invention are also included peptides derived or obtained from any of the peptides disclosed above, preferably incorporating one or more conservative substitutions, as long as they retain or show or improve at least one of the activities mentioned above (and, preferably, all of the activities mentioned above for the peptides of the present invention).

In addition, also included within the scope of the present invention are peptides with a 70% percentage identity, preferably 80%, more preferably 90%, more preferably 95%, even more preferably 99% percentage identity with any of the specific sequences mentioned above as long as they retain or show or improve at least one of the activities mentioned above (and, preferably, all of the activities mentioned above for the peptides of the present invention).

As it is directly derivable from the above, it is contemplated that the peptide of the present invention is a linear peptide or a cyclic peptide.

In an embodiment, which can be combined with any of the above embodiments, the peptide is cyclic. In this case, the first and the last amino acid of the peptide of the present invention (in accordance with the above explanations) are bound. Therefore, appropriate modification to any of the above embodiments has to be done (for example, R$_1$ and R$_2$ may be absent as the amino acids to which said moieties are bound, are now bound to each other).

In a preferred embodiment, which can be combined with any of the above embodiments, the peptide is linear.

The peptides of the present invention may be synthesized and produced by any means known in the state of the art. For example, they may be synthesized and produced by chemical synthesis (preferably, by means of solid phase peptide synthesis), expressing said peptides in cell cultures or by means of transgenic production of the peptide in plants or animals. In addition, the peptides of the present invention may be purified by any means known in the state of the art.

In a preferred embodiment, which can be combined with any of the embodiments disclosed above, the peptide of the present invention is suited or adapted to be applied by means of iontophoresis, more preferably, in the face of a subject (preferably, a human).

In a further preferred embodiment, which can be combined with any of the embodiments disclosed above, the peptide of the present invention is suited or adapted to be applied subcutaneously, more preferably, in the face of a subject (preferably, a human).

In another preferred embodiment, which can be combined with any of the embodiments disclosed above, the peptide of the present invention is suited or adapted to be applied topically (preferably, in the form of a cream), more preferably, in the face of a subject (preferably, a human).

As it can be directly derived from the examples included below, the peptides of the present invention provide for a broad spectrum of activities which make them useful in cosmetics for the prevention and/or treatment of skin aging and inflammaging:

- Protection of telomeres.
- Lengthening of telomeres.

- Protection against inflammation or anti-inflammatory activity.
- Antioxidative activity.
- Protection against heat.

Therefore, the peptides of the present invention provide for a broad-spectrum activity for the prevention and/or treatment of inflammaging and skin aging and solve the problems present in the state of the art and mentioned above.

In addition, the peptides of the present invention are also useful in medicine due to the above-mentioned activities (especially anti-inflammatory and antioxidative activities). Said activities demonstrate that the peptides of the present invention are useful, for example, for the treatment of age-related diseases, inflammatory diseases and/or diseases related with oxidative stress.

Moreover, due to the above activities (especially anti-inflammatory and antioxidative activities), which appear demonstrated in the examples included below, the peptides of the present invention are also useful in the food industry, preferably as nutraceuticals.

In a second aspect, the present invention refers to a composition comprising a peptide of the present invention.

The peptide of the present invention is as disclosed above in the first aspect of the present invention.

The composition of the present invention is preferably a cosmetic composition. Hence, in this embodiment, the cosmetic composition of the present invention comprises a cosmetically effective amount of the at least one peptide of the present invention, more preferably the cosmetic composition of the present invention comprises from 0.0001% (weight/volume in g/100 mL, hereinafter, w/v) to 0.05% (w/v) of a peptide of the present invention. In a most preferred embodiment, the cosmetic composition of the present invention comprises from 0.0001% (w/v) to 0.005% (w/v) of a peptide of the present invention, more preferably, from 0.0005% (w/v) y 0.0015% (w/v).

It is contemplated that the cosmetic composition of the present invention also comprises at least one additional cosmetic ingredient. Said additional cosmetic ingredient can be at least one excipient and/or at least one additional cosmetic active ingredient. Said additional cosmetic ingredient can be any cosmetic ingredient known in the state of the art as long as it does not affect or it does not affect in an unacceptable manner the activity of the peptides of the present invention.

In another embodiment, the composition of the present invention is a pharmaceutical composition.

It is contemplated that the pharmaceutical composition of the present invention also comprises at least one additional pharmaceutical ingredient. Said additional pharmaceutical ingredient can be at least one excipient and/or at least one additional pharmaceutical active ingredient. Said additional pharmaceutical ingredient can be any cosmetic ingredient known in the state of the art as long as it does not affect or it does not affect in an unacceptable manner the activity of the peptides of the present invention.

In a further embodiment, the composition of the present invention is a food composition.

It is contemplated that the food composition of the present invention also comprises at least one food ingredient. Said additional food ingredient can be any food ingredient known in the state of the art as long as it does not affect or it does not affect in an unacceptable manner the activity of the peptides of the present invention.

Preferably, the food composition of the present invention is a nutraceutical.

In an embodiment, the composition of the present invention consists essentially of peptides of the present invention.

In another embodiment, the composition of the present invention consists of peptides of the present invention.

In a third aspect, the present invention refers to the use as a cosmetic of a peptide of the present invention or of a composition of the present invention, in a subject in need of the treatment.

The peptide of the present invention is as disclosed above in the first aspect of the present invention.

The composition of the present invention is as disclosed above in the second aspect of the present invention.

The peptide or the composition of the present invention are used in a cosmetically effective amount or quantity.

The composition of the present invention is, preferably, a cosmetic composition as disclosed above in the second aspect of the present invention.

In a preferred embodiment, the use as a cosmetic is to prevent, treat and/or reduce skin aging, more preferably, reduce, prevent and/or eliminate signs of skin aging.

Preferably, the signs of skin aging are wrinkles, skin roughness (preferably, wrinkle roughness), looser skin or sagging skin, more preferably, wrinkles, skin roughness, non-inflammatory looser skin or non-inflammatory sagging skin, more preferably, wrinkles, even more preferably facial wrinkles. Said wrinkles can be chronological wrinkles or expression (muscle-derived) wrinkles.

Non-inflammatory looser skin is equivalent to cosmetic looser skin, this is, non-pathological (non-medical condition).

Non-inflammatory sagging skin is equivalent to cosmetic sagging skin, this is, non-pathological (non-medical condition).

In another preferred embodiment, the use as a cosmetic is to prevent, treat and/or reduce inflammaging, more preferably skin inflammaging.

In this third aspect of the present invention, the subject in need of the treatment is preferably a mammal, more preferably, a human.

In this third aspect of the present invention, it is also contemplated that the use as a cosmetic is for the improvement of skin firmness and/or elasticity.

In a fourth aspect, as noted above, the present invention refers to the cosmetic use of a peptide of the present invention or a composition of the present invention in a subject in need of the treatment.

Preferably, in this fourth aspect of the present invention, the cosmetic use is to prevent, treat and/or reduce skin aging, more preferably, reduce, prevent and/or eliminate signs of skin aging.

In another preferred embodiment, the cosmetic use is to prevent, treat and/or reduce inflammaging, more preferably skin inflammaging. Inflammaging is cosmetic inflammaging, preferably cosmetic skin inflammaging.

In this fourth aspect of the present invention it is also contemplated that the cosmetic use is for the improvement of skin firmness and/or elasticity.

The peptide of the present invention is as disclosed above in the first aspect of the present invention.

The composition of the present invention is as disclosed above in the second aspect of the present invention.

The composition of the present invention is, preferably, a cosmetic composition as disclosed above in the second aspect of the present invention.

The signs of skin aging and the subject in need of the treatment are as explained above in the third aspect of the present invention.

In addition, all the embodiments explained above in the third aspect of the present invention are directly applicable to this fourth aspect of the present invention, with the appropriate adaptations.

In a fifth aspect, the present invention refers to a method for the prevention and/or treatment of skin aging or of inflammaging in a subject comprising the administration of a peptide of the present invention or a composition of the present invention to the subject.

The peptide of the present invention is as disclosed above in the first aspect of the present invention.

The composition of the present invention is as disclosed above in the second aspect of the present invention.

The composition of the present invention is, preferably, a cosmetic composition as disclosed above in the second aspect of the present invention.

The signs of skin aging, inflammaging and the subject are as explained above in the third aspect of the present invention.

In addition, all the embodiments explained above in the third and fourth aspects of the present invention are directly applicable to this fifth aspect of the present invention, with the appropriate adaptations.

In a sixth aspect, the present invention refers to a composition or a peptide of the present invention for use as a medicament.

The peptide of the present invention is as disclosed above in the first aspect of the present invention.

The composition of the present invention is as disclosed above in the second aspect of the present invention.

The peptide or the composition of the present invention are used in a pharmaceutically effective amount or quantity.

The composition of the present invention is, preferably, a pharmaceutical composition as disclosed above in the second aspect of the present invention.

In this sixth aspect of the present invention, the composition or peptide of the present invention is administered to a subject in need of the treatment. Preferably, the subject in need of the treatment is a mammal, more preferably, a human.

In a preferred embodiment, the use as a medicament is for use in the prevention, amelioration and/or treatment of an age-related disease, an inflammatory disease and/or a disease related to oxidative stress.

In a preferred embodiment, the use as a medicament is for the prevention, amelioration and/or treatment of an inflammatory disease.

Preferably, the inflammatory disease is a chronic inflammatory disease, more preferably rheumatoid arthritis, inflammatory bowel disease, psoriasis or palmoplantar pustulosis.

Also preferably, the inflammatory disease is rosacea, atopic skin, psoriasis or acne.

It is also contemplated that the inflammatory disease is inflammaging, more preferably, non-cosmetic inflammaging, this is, pathological inflammaging.

It is also contemplated that the inflammatory disease is inflammatory looser skin or inflammatory sagging skin (this is, pathological looser skin or pathological sagging skin).

In a further preferred embodiment, the use as a medicament is for the prevention, amelioration and/or treatment of a disease related with oxidative stress.

Preferably, the disease related with oxidative stress is selected from cardiovascular diseases (CVDs) (preferably, atherosclerosis), chronic obstructive pulmonary disease, acute or chronic kidney disease, clinical frailty, neurodegenerative diseases (preferably, AD, Parkinson's disease (PD), Sarcopenia, Huntington's disease (HD), amyotrophic lateral sclerosis (ALS) and vascular dementia), macular degeneration (MD), biliary diseases cancer, biliary cirrhosis, cholangitis, osteoarthritis and diabetes.

In a seventh aspect, the present invention refers to the use of the composition or a peptide of the present invention for the manufacture of a medicament for the prevention, amelioration and/or treatment of an age-related disease, an inflammatory disease or a disease related to oxidative stress.

The peptide of the present invention is as disclosed above in the first aspect of the present invention.

The composition of the present invention is as disclosed above in the second aspect of the present invention.

The peptide or the composition of the present invention are used in a pharmaceutically effective amount or quantity.

The composition of the present invention is, preferably, a pharmaceutical composition as disclosed above in the second aspect of the present invention.

The age-related disease, the inflammatory disease and the disease related to oxidative stress are as disclosed above in the sixth aspect of the present invention.

In this seventh aspect of the present invention, the composition or peptide of the present invention is administered to a subject in need of the treatment. Preferably, the subject in need of the treatment is a mammal, more preferably, a human.

In an eighth aspect, the present invention refers to a method for the prevention, amelioration and/or treatment of an age-related disease, an inflammatory disease or a disease related to oxidative stress, comprising administering a peptide or a composition of the present invention to a subject in need thereof.

The peptide of the present invention is as disclosed above in the first aspect of the present invention.

The composition of the present invention is as disclosed above in the second aspect of the present invention.

The peptide or the composition of the present invention are used in a pharmaceutically effective amount or quantity.

The composition of the present invention is, preferably, a pharmaceutical composition as disclosed above in the second aspect of the present invention.

The age-related disease, the inflammatory disease and the disease related to oxidative stress are as disclosed above in the sixth aspect of the present invention.

Preferably, the subject in need of the treatment is a mammal, more preferably, a human.

In a ninth aspect, the present invention refers to a nutraceutical composition comprising a peptide of the present invention.

The peptide of the present invention is as disclosed above in the first aspect of the present invention.

In a final aspect, the present invention refers to the use as a nutraceutical of a peptide of the present invention or of a composition of the present invention.

The peptide of the present invention is as disclosed above in the first aspect of the present invention.

The composition of the present invention is as disclosed above in the second aspect of the present invention. More preferably, the composition of the present invention isa food composition as explained in the second aspect of the present invention.

The activities mentioned above and demonstrated in the experiments included below, especially the anti-inflammatory and antioxidative activities, demonstrate the usefulness of the peptides and compositions of the present invention as nutraceuticals.

SEQUENCE LISTING FREE TEXT

SEQ ID NO: 1

<223> Artificially synthesized sequence
<223> Xaa is a small amino acid
<223> Xaa is selected from polar uncharged or aromatic amino acids
<223> Xaa is absent or selected from aromatic amino acids
<223> Xaa is selected from negatively charged amino acids
<223> Xaa is selected from apolar amino acids
<223> Xaa is selected from Asn or Leu
<223> Xaa is selected from negatively charged amino acid
<223> Xaa is absent or selected from aromatic amino acids

SEQ ID NO: 2
<223> Artificially synthesized sequence

SEQ ID NO: 3
<223> Artificially synthesized sequence

To allow a better understanding, the present invention is described in more detail below with reference to the enclosed drawings, which are presented by way of example, and with reference to illustrative and non-limitative examples. In the figures: * means p<0.05; ** means p< 0.01; *** means p<0.001.

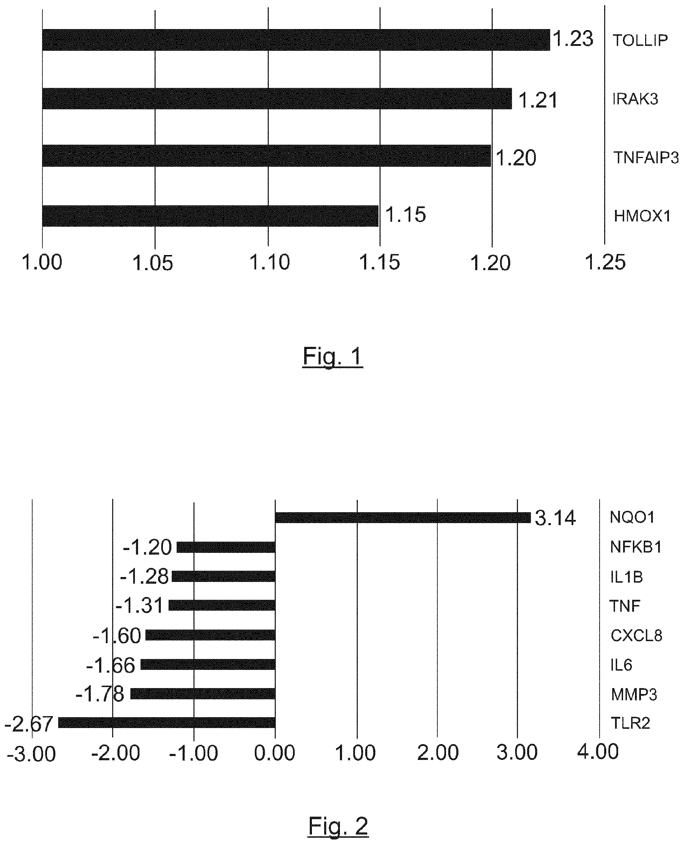

Figure 1 shows the gene expression results obtained in example 2 for the treatment of HEKa cells with peptide SEQ ID NO: $2\text{-}NH_2$ at 6 h with 0.01 mg/mL of the peptide. Each of the bars refers to one gene, from top to bottom: TOLLIP, IRAK3, TNFAIP3 and HMOX1. Bars growing towards the right (this is, with a positive value) show an upregulation of the gene.

Figure 2 shows the gene expression results obtained in example 2 for the treatment of HEKa cells with peptide SEQ ID NO: $2\text{-}NH_2$ at 24 h with 0.01 mg/mL of the peptide. Each of the bars refers to one gene, from top to bottom: NQO1, NFKB1, IL1B, TNF, CXCL8, IL6, MMP3 and TLR2. Bars growing towards the left (this is, with a negative value) show a downregulation of the gene. Bars growing towards the right (this is, with a positive value) show an upregulation of the gene.

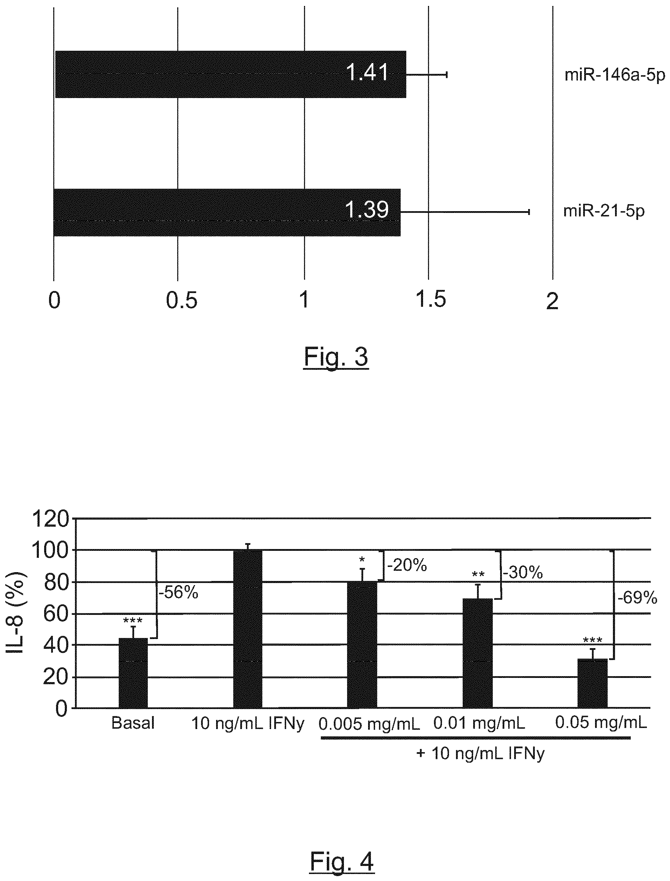

Figure 3 shows the miRNA expression results obtained in example 3 for the treatment of HEKa cells with peptide SEQ ID NO: $2\text{-}NH_2$ at 24 h with 0.01 mg/mL of the peptide. Each of the bars refers to one miRNA, from top to bottom: miR-146a-5p and miR-21-5p. Bars growing towards the right (this is, with a positive value) show an upregulation of the gene.

Figure 4 shows the results obtained for IL-8 secretion or release under inflammatory conditions (except the untreated or basal sample) in example 4 for the treatment of HaCaT keratinocytes with peptide SEQ ID: $2\text{-}NH_2$. The results in this figure are shown with regard to the positive control sample (cells treated with IFNy at 10ng/mL), for which sample secretion of IL-8 is set as 100%. The columns or bars in the x-axis refer to the different experimental treatment groups, from left to right: negative control (basal), positive control, cells treated with 10 ng/mL IFNy and 0.005 mg/mL, 0.01 mg/mL or 0.05 mg/mL of peptide SEQ ID NO: $2\text{-}NH_2$. The y-axis refers to the percentage of IL-8 secreted with regard to the positive control (stablished as 100%).

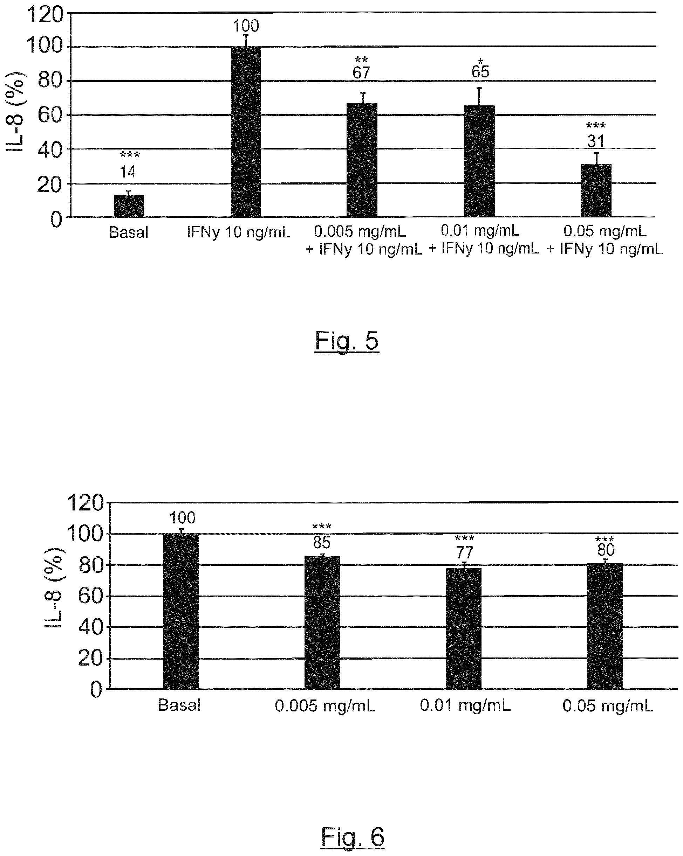

Figure 5 shows the results obtained for IL-8 secretion or release under inflammatory conditions (except the untreated or basal sample) in example 4 for the treatment of HaCaT keratinocytes with peptide SEQ ID: $3\text{-}NH_2$. The results in this figure are shown with regard to the positive control sample (cells treated with IFNy at 10ng/mL), for which sample secretion of IL-8 is set as 100%. The columns or bars in the x-axis refer to the different experimental treatment groups, from left to right: negative control (basal), positive control, cells treated with 10 ng/mL IFNy and 0.005 mg/mL, 0.01 mg/mL or 0.05 mg/mL of peptide SEQ ID NO: $3\text{-}NH_2$. The y-axis refers to the percentage of IL-8 secreted with regard to the positive control (stablished as 100%).

Figure 6 shows the results obtained for IL-8 secretion or release under non-inflammatory conditions in example 4 for the treatment of HaCaT keratinocytes with peptide SEQ ID: $2\text{-}NH_2$. The results in this figure are shown with regard to the basal state (untreated cells), for which sample secretion of IL-8 is set as 100%. The columns or bars in the x-axis refer to the different experimental treatment groups, from left to right: negative control (basal), cells treated with 0.005 mg/mL, 0.01 mg/mL or 0.05 mg/mL of peptide SEQ ID NO: $2\text{-}NH_2$. The y-axis refers to the percentage of IL-8 secreted with regard to the basal state (stablished as 100%).

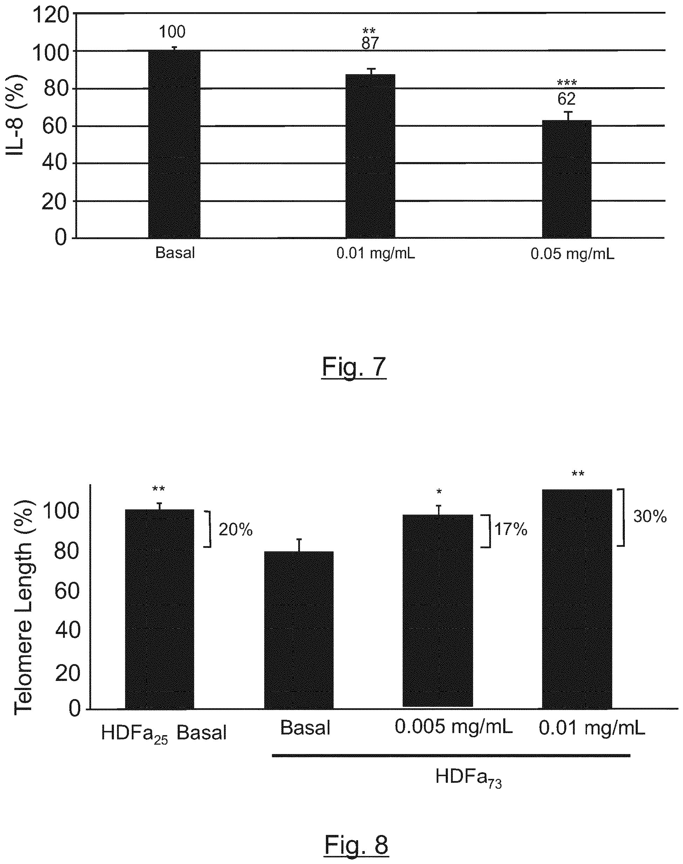

Figure 7 shows the results obtained for IL-8 secretion or release under non-inflammatory conditions in example 4 for the treatment of HaCaT keratinocytes with peptide SEQ ID: $3\text{-}NH_2$. The results in this figure are shown with regard to the basal state (untreated cells), for which sample secretion of IL-8 is set as 100%. The columns or bars in the x-axis refer to the different experimental treatment groups, from left to right: negative control (basal), cells treated with 0.01 mg/mL or 0.05 mg/mL of peptide SEQ ID NO: $3\text{-}NH_2$. The y-axis refers to the percentage of IL-8 secreted with regard to the basal state (stablished as 100%).

Figure 8 shows the results obtained for telomere length in example 5 for the treatment of HDFa cells with peptide SEQ ID: $2\text{-}NH_2$. The results are expressed as % of telomere length with regard to the telomere length of HDFa from a 25-year-old subject ($HDFa_{25}$), which is stablished as 100%. The columns or bars in the x-axis refer to the different experimental treatment groups, from left to right: Untreated $HDFa_{25}$ cells, Untreated $HDFa_{73}$ cells and $HDFa_{73}$ cells (HDFa from a 73-year-old subject) treated with 0.005 mg/mL or 0.01 mg/mL, respectively. The y-axis refers to the percentage of telomere length with regard to the untreated $HDFa_{25}$ cells (stablished as 100%).

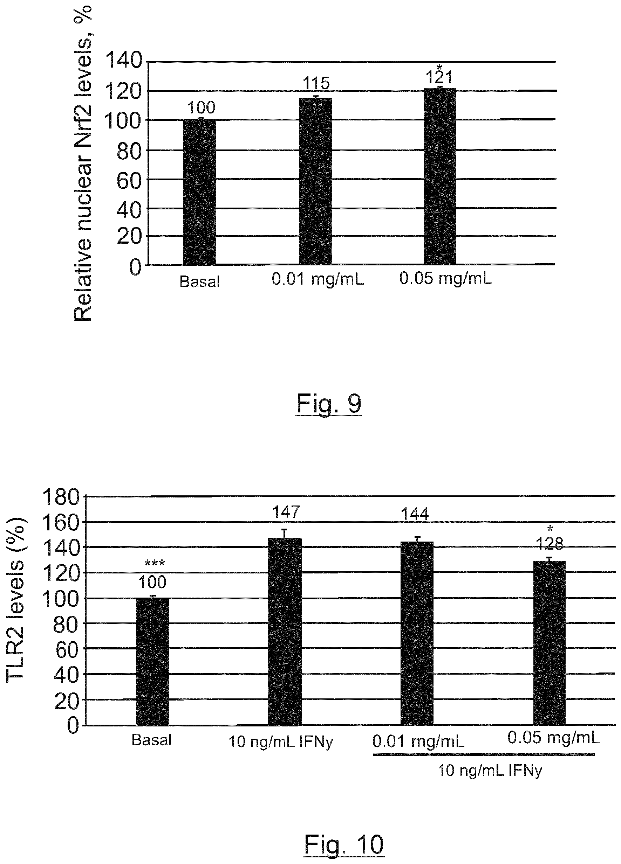

Figure 9 shows the results obtained for Nrf2 translocation into the nuclei in example 6 for the treatment of HEKa cells with peptide SEQ ID NO: $2\text{-}NH_2$. The results are expressed as % of nucleolar Nrf2 levels in comparison with the nucleolar Nrf2 levels of the untreated cells (negative control - basal state), which is set as 100%. The columns or bars in the x-axis refer to the different experimental treatment groups, from left to right: untreated cells and cells treated with 0.01 and 0.05 mg/mL of the peptide of the present invention. The y-axis refers to the nucleolar Nrf2 levels expressed as a % with regard to those of the untreated cells which are stablished as 100%.

Figure 10 shows the results obtained for the levels of TLR2 in example 7 for the treatment of HEKa cells with peptide SEQ ID NO: 2-NH$_2$. The results are expressed as % of the levels of TLR2 in comparison with the untreated cells (negative control - basal state), which is set as 100%. The columns or bars in the x-axis refer to the different experimental treatment groups, from left to right: untreated cells; cells treated with 10 ng/mL of IFNγ; and cells treated with 10 ng/mL of IFNy and 0.01 or 0.05 mg/mL of the peptide of the present invention, respectively. The y-axis refers to levels of TLR2 expressed as a % with regard to those of the untreated cells which are stablished as 100%.

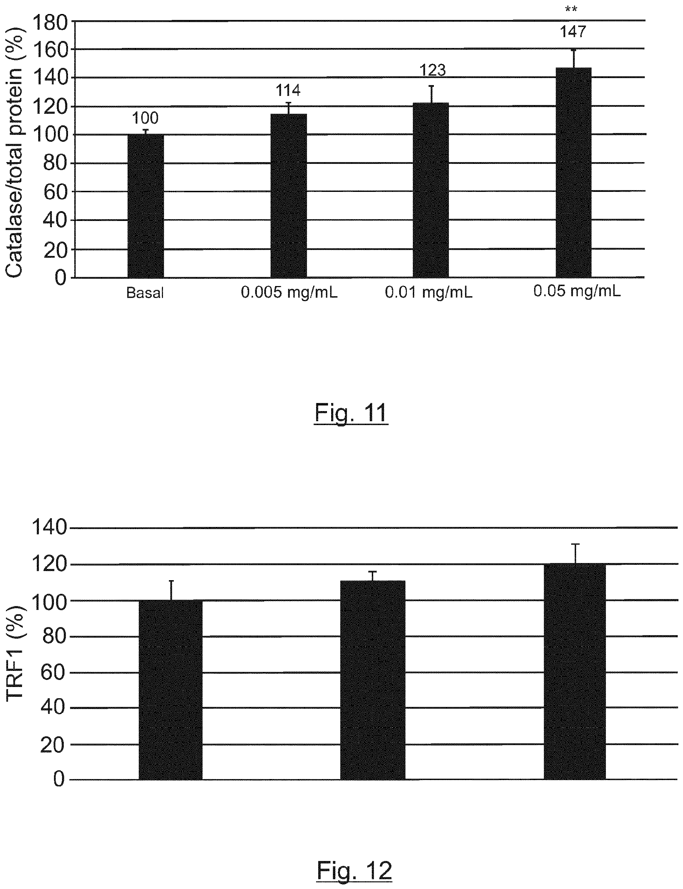

Figure 11 shows the results obtained for catalase levels or synthesis in example 8 for the treatment of HEKa cells with peptide SEQ ID NO: 2-NH$_2$. The results are expressed as catalase levels with regard to the total protein quantity of a sample and as a percentage with regard to the value obtained for the basal state (untreated cells) which is stablished as 100%. The columns or bars in the x-axis refer to the different experimental treatment groups, from left to right: untreated cells (basal state); and cells treated with 0.005 mg/mL, 0.01 mg/mL or 0.05 mg/mL of the above-mentioned peptide. The y-axis refers to the percentage of the levels of catalase/total protein with regard to the basal state which is stablished as 100%.

Figure 12 shows the results obtained for TRF1 production (TRF1 levels as measured by means of the measured intensity) in example 9 with the treatment of CHA9.3 cells with peptides SEQ ID NO: 2-NH$_2$ and SEQ ID NO: 3-NH$_2$. Results are normalized with regard to the negative control (cells only treated with the vehicle, this is, DMSO). The columns or bars in the x-axis refer to the different experimental treatment groups, from left to right: negative control (cells treated with DMSO), cells treated with 0.063 mg/mL of peptide SEQ ID NO: 3-NH$_2$, cells treated with 0.125 mg/mL of peptide SEQ ID NO: 2-NH$_2$. The y-axis refers to the TRF1 intensity obtained with regard to the negative control (intensity of TRF1 is stablished as 100% for the negative control group and the value for the rest of the treatment groups is stablished by comparison with said negative control).

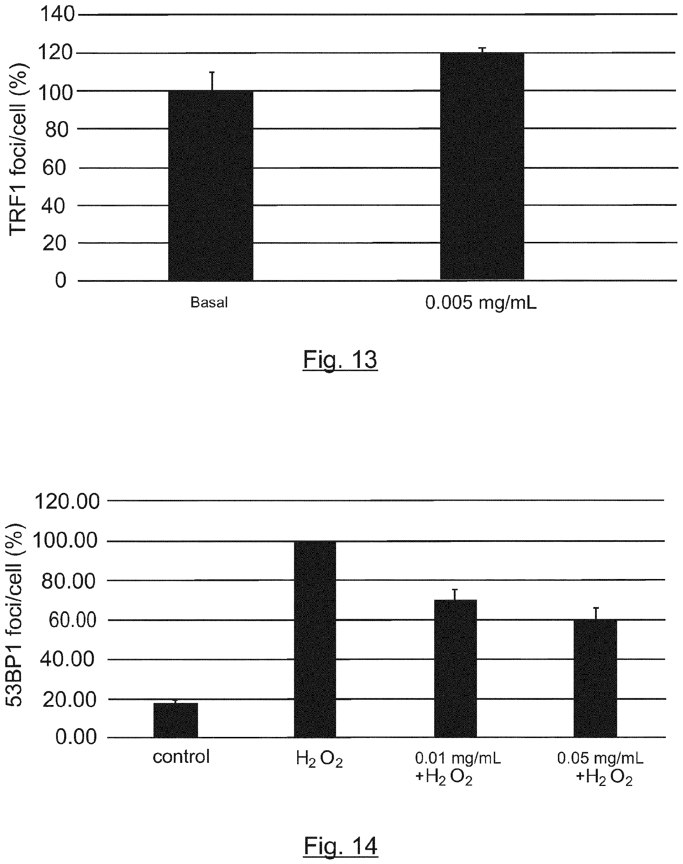

Figure 13 shows the results obtained for TRF1 foci per cell in example 10 with the treatment of HEKa cells with peptide SEQ ID NO: 2-NH$_2$. Results are normalized with regard to the negative control (basal state, cells not treated with the peptide of the present invention). The columns or bars in the x-axis refer to the different experimental treatment groups, from left to right: negative control (basal state) and cells treated with 0.005 mg/mL of peptide SEQ ID NO: 2-NH$_2$. The y-axis refers to the TRF1 foci per cell with regard to the negative control (number of foci of TRF1 per cell is stablished as 100% for the negative control group and the value for the rest of the treatment groups is stablished by comparison with said negative control).

Figure 14 shows the results obtained for DNA protection (measured on the basis of the foci of 53BP1 per cell) obtained in example 10 with the treatment of HEKa cells with peptide SEQ ID NO: 2-NH$_2$. Results are normalized with regard to the positive control (cells treated only with H$_2$O$_2$). The columns or bars in the x-axis refer to the different experimental treatment groups, from left to right: negative control (basal state, non-treated cells), positive control (cells treated only with H$_2$O$_2$), cells treated with H$_2$O$_2$ and 0.01 mg/mL or 0.05 mg/mL of the peptide of the present invention. The y-axis refers to the 53BP1 foci per cell with regard to the positive control (number of foci of 53BP1 per cell is stablished as 100% for the positive control group and the value for the rest of the treatment groups is stablished by comparison with said negative control).

## EXAMPLES

Abbreviations:

The abbreviations used in the present text for amino acids follow the 1983 IUPAC-IUB Joint Commission on Biochemical Nomenclature recommendations outlined in Eur. J. Biochem. (1984) 138:937.

For the examples:

Asn, Asparagine; Asp, aspartic acid; BSA, Bovine Serum Albumin; cDNA, Complementary DNA; C-terminal, carboxy-terminal; DAPI, 4',6-diamidino-2-phenylindole; DCM, dichloromethane; DIPCDI, N,N'-diisopropylcarbodiimide; DMEM, Dulbecco's Modified Eagle Medium; DMF, N,N-dimethylformamide; DMSO, Dimethyl sulphoxide; DNA, Deoxyribonucleic acid; equiv, equivalent; ESI-MS, electrospray ionization mass spectrometry; FBS, Fetal Bovine Serum; Fmoc, 9-fluorenylmethyloxycarbonyl; Glu, Glutamic acid; Gly, Glycine; HOBt, 1-hydroxybenzotriazole; HPLC, high performance liquid chromatography; IFN-y, Interferon gamma; Leu, Leucine; LSGS, Low Serum Growth Supplement; MBHA, p-methylbenzhydrylamine; Me, methyl; MeCN, acetonitrile; MeOH, methanol; N-terminal, amino-terminal; PBS, Phosphate Borate Saline; qPCR, real-time polymerase chain reaction; RH, Relative Humidity; RIPA, Radioimmunoprecipitation assay buffer; RNA, Ribonucleic acid; RT, room temperature; RT-qPCR, Quantitative reverse transcription Polymerase Chain Reaction; tBu, *tert*-butyl; TFA, trifluoroacetic acid; TIS, triisopropylsilane; Trt, triphenylmethyl or trityl; Tyr, tyrosine.

Regarding the chemical synthesis procedures included in the examples, it is noted that all synthetic processes were carried out in polypropylene syringes fitted with porous polyethylene discs or Pyrex® reactors fitted with porous plates. All the reagents and solvents were synthesis quality and were used without any additional treatment. The solvents and soluble reagents were removed by suction. The Fmoc group was removed with piperidine-DMF (2:8, volume/volume, v/v) (at least 1x1 min, 2x10 min, 5 mL/g resin) (Lloyd Williams P. et al., Chemical Approaches to the Synthesis of Peptides and Proteins, CRC, 1997, Boca Raton (Fla., USA)). Washes between stages of deprotection, coupling, and, again, deprotection, were carried out with DMF (3×1 min) and DCM (3×1 min) each time using 10 mL solvent/g resin. Coupling reactions were performed with 3 mL solvent/g resin. The control of the couplings was performed by carrying out the ninhydrin test (Kaiser E. et al., Anal. Biochem., 1970, 34: 595598). All synthetic reactions and washes were carried out at RT.

**Example 1.** Synthesis and preparation of the peptides.

**a) Obtaining Fmoc-AA1-AA2-AA3-AA4-AA5-AA6-AA7-Rink-MBHA-resin, wherein AA1 is L-Gly; AA2 is L-Tyr; AA3 is L-Tyr; AA4 is L-Glu; AA5 is L-Leu; AA6 is L-Asn and AA7 is L-Asp.**

Weights were normalized. 4.8 g (2.5 mmol) of the Fmoc-Rink-MBHA resin with a functionalization of 0.52 mmol/g were treated with piperidine-DMF according to the described general protocol known in the state of the art in order to remove the Fmoc group. 3.08 g of Fmoc-L-Asp(tBu)-OH (7.5 mmol; 3 equiv) were incorporated onto the deprotected resin in the presence of DIPCDI (1.17 mL; 7.5 mmol; 3 equiv) and HOBt (1.01 g; 7.5 mmol; 3 equiv) using DMF as a solvent for one hour.

The resin was then washed as described in the general methods known in the state of the art and the deprotection treatment of the Fmoc group was repeated to couple the next amino acid. Following the previously described protocols 4.45 g of Fmoc-L-Asn(Trt)-OH (7.5 mmol; 3 equiv); subsequently 2.65 g of Fmoc-L-Leu-OH (7.5 mmol; 3 equiv); subsequently 3.19 g of Fmoc-L-Glu(OtBu)-OH (7.5 mmol; 3 equiv); subsequently 3.44 g Fmoc-L-Tyr(tBu)-OH (7.5 mmol; 3 equiv), subsequently 3.44 g Fmoc-L-Tyr(tBu)-OH (7.5 mmol; 3 equiv) and subsequently 2.23 g of Fmoc-L-Gly-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.

**b) Obtaining Fmoc-AA1-AA2-AA3-AA4-AA5-AA6-AA7-Rink-MBHA-resin, wherein AA1 is L-Gly; AA2 is L-Asn; AA3 is L-Glu; AA4 is L-Leu; AA5 is L-Leu; AA6 is L-Asp and AA7 is L-Tyr.**

Weights were normalized. 4.8 g (2.5 mmol) of the Fmoc-Rink-MBHA resin with a functionalization of 0.52 mmol/g were treated with piperidine-DMF according to the described general protocol known in the state of the art in order to remove the Fmoc group. 3.44 g of Fmoc-L-Tyr(tBu)-OH (7.5 mmol; 3 equiv) were incorporated onto the deprotected resin in the presence of DIPCDI (1.17 mL; 7.5 mmol; 3 equiv) and HOBt (1.01 g; 7.5 mmol; 3 equiv) using DMF as a solvent for one hour.

The resin was then washed as described in the general methods known in the state of the art and the deprotection treatment of the Fmoc group was repeated to couple the next amino acid. Following the previously described protocols 3.08 g of Fmoc-L-Asp(tBu)-OH (7.5 mmol; 3 equiv); subsequently 2.65 g of Fmoc-L-Leu-OH (7.5 mmol; 3 equiv); subsequently 2.65 g of Fmoc-L-Leu-OH (7.5 mmol; 3 equiv): subsequently 3.19 g of Fmoc-L-Glu(OtBu)-OH (7.5 mmol; 3 equiv); subsequently 4.45 g of Fmoc-L-Asn(Trt)-OH (7.5 mmol; 3 equiv) and subsequently 2.23 g of Fmoc-L-Gly-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.

Using the synthesis procedures mentioned above, with the required selection of amino acids, the following sequences were synthesized:

- $R_1$-Gly-Tyr-Tyr-Glu-Leu-Asn-Asp-$R_2$ ($R_1$-SEQ ID NO: 2-$R_2$); or
- $R_1$-Gly-Asn-Glu-Leu-Leu.Asp-Tyr-$R_2$ ($R_1$-SEQ ID NO: 3-$R_2$)

**c) Removal of Fmoc N-Terminal protective group of the peptides synthesized in accordance with a) or b).**

The N-terminal Fmoc group of the peptidyl resins obtained in a) or b) above, was deprotected with 20% (volume/volume, hereinafter v/v) piperidine in DMF ($1\times1$ min+$2\times10$ min) (Lloyd Williams P. et al. (1997) Chemical Approaches to the Synthesis of Peptides and Proteins. CRC, Boca Raton (Fla., USA)). The peptidyl resins were washed with DMF ($5\times1$ min), DCM ($4\times1$ min), and dried under vacuum.

**d) Cleavage process from the polymeric support of the peptidyl resins obtained in accordance with c).**

Weights were normalized. 200 mg of the dried peptidyl resin obtained in c) were treated with 5 mL of TFA/TIS/$H_2O$ (90:5:5, in volume) for 2 hours at room temperature under stirring. The filtrates were collected and precipitated using 50 mL (8 to 10-fold) of cold diethyl ether. The ethereal solutions were evaporated to dryness at reduced pressure and room temperature, the precipitates were redissolved in 50% (v/v) MeCN in $H_2O$ and lyophilized.

**e) Characterization of the peptides synthesized and prepared in accordance with d)**.

HPLC analysis of the peptides obtained in accordance with d) was carried out with a Shimadzu equipment (Kyoto, Japan) using a reverse-phase column (150x4.6 mm, XBridge Peptide BEH C18, 3.5 $\mu$m, Waters, USA) in gradients of MeCN (+0.036% (v/v) TFA) in $H_2O$ (+0.045% (v/v) TFA) at a flow rate of 1.25 mL/min and detection was carried out at 220 nm. All peptides showed a purity exceeding 80%. The identity of the peptides obtained was confirmed by ESI-MS in a Water ZQ 4000 detector using MeOH as the mobile phase and a flow rate of 0.2 mL/min. Results obtained demonstrated that peptides SEQ ID NO: 2- $NH_2$ and SEQ ID NO: 3- $NH_2$, were correctly and effectively synthesized.

**Example 2. Analysis of the expression inflammation-related genes**

For this example, peptide SEQ ID NO: 2-$NH_2$ was prepared in accordance with example 1.

A stock solution of the peptide was prepared in DMSO at 50 mg/mL. This stock was further diluted to obtain the final working concentrations of 0.01 mg/mL in complete medium (Dermal Cell Basal Medium (DCBM) supplemented with keratinocyte Growth Kit and 0.1% Penicillin/Streptomycin). Untreated cells were used as negative control sample (basal sample).

The relevant genes for which the expression was measured were: TOLLIP (toll interacting protein), IRAK3 (interleukin 1 receptor associated kinase 3), TNFAIP3 (TNF alpha induced protein 3), HMOX1 (heme oxygenase 1), NQO1 (NAD(P)H quinone dehydrogenase 1), NFKB1 (nuclear factor kappa B subunit 1), TNF (Tumoral necrosis factor), IL1B (interleukin 1 beta), IL6 (Interleukin 6), CXCL8 (C-X-C motif chemokine ligand 8), MMP3 (matrix metallopeptidase 3), TLR2 (toll like receptor 2). The primers used for each of the genes were commercially available TaqMan Gene Expression Assay probes. The experiment was performed in vitro in HEKa cells, one concentration of the peptide was tested (0.01 mg/mL) at 6h and 24h.

Briefly, human epidermal keratinocytes from adult (HEKa) were seeded in 6-well plates at a density of 300,000 cells/well and maintained at standard culture conditions (DCBM medium supplemented with keratinocyte Growth Kit and 0.1% Penicillin/Streptomycin; 37°C, 95% Relative Humidity, 5% $CO_2$) for 24 hours. Next, HEKa were incubated in the presence of 0.01 mg/mL of the peptide for 6 or 24 h.

Cells were finally lysed and RNA extraction was performed using Qiagen RNeasy Mini kit following manufacturer's instructions. Purified RNAs were used to generate the corresponding cDNAs by reverse transcription which served as templates for amplification. RT-qPCR was performed with the panel of TaqMan assay probes specified above and 2x gene expression Master Mix using StepOne plus Real-Time PCR instrument. Amplification included 40 cycles of: 15 seconds at 95°C (denaturation) and 1 minute at 60°C (Annealing and extension).

The obtained data were analysed using the $\Delta\Delta$Ct method, which provides the target gene expression values as fold changes in the treated sample compared with an untreated basal sample. All samples were normalized with the relative expression of a housekeeping gene GAPDH (Glyceraldehyde 3-phosphate dehydrogenase).

The steps for analysis included:

1. Calculate the average Ct for each condition
2. Calculate the $\Delta$CT test sample and the $\Delta$CT calibrator sample
3. Calculate the $\Delta\Delta$CT: $\Delta\Delta$CT = $\Delta$CT test sample - $\Delta$CT calibrator sample
4. Obtain ratio by $2^{-\Delta\Delta CT}$

Gene modulations with a ratio (fold change vs. basal samples) >1.15 or <(-1.15) are considered of potential biological importance.

The results obtained appear summarized in Figures 1 and 2, which show that the peptide SEQ ID NO: 2-NH$_2$ (as an example of the peptides of the present invention) was able to modulate the expression of inflammation-related genes, upregulating the anti-inflammatory ones and downregulating the pro-inflammatory ones. More precisely, after 6 h, HEKa cells treated with the peptide of the present invention showed an upregulation of genes TNFAIP3, TOLLP, HMOX1 and IRAK3, whereas after 24 h treatment with the peptide of the present invention NQO1 was upregulated and NFKB1, IL1B, TNF, CXCL8, IL6, MMP3 and TLR2 genes were downregulated. All these results validate or demonstrate the usefulness of the peptides of the present invention against inflammation and, hence, for the prevention, reduction and/or treatment of inflammaging and skin aging. In addition, all these results also demonstrate the usefulness of the peptides of the present invention for the treatment of age-related diseases and inflammatory diseases, as explained above. Also, this anti-inflammatory activity demonstrates the usefulness of the peptides of the present invention as nutraceuticals.

**Example 3. Analysis of the expression of inflammation-related miRNAs.**

For this example, peptide SEQ ID NO: 2-NH$_2$ was prepared in accordance with example 1.

A stock solution of the peptide was prepared in DMSO at 50 mg/mL. This stock was further diluted to obtain the final working concentrations of 0.01 mg/mL in complete medium (Dermal Cell Basal Medium (DCBM) supplemented with keratinocyte Growth Kit and 0.1% Penicillin/Streptomycin). Untreated cells were used as negative control sample (basal sample).

The relevant miRNAs for which the expression was measured were: miR-21-5p and miR-146a-5p. During the last years, microRNAs (miRNAs) has been identified as another key element in aging and inflammaging. miR-21-5p and miR-146a-5p are involved in the modulation of NF-κB and NLRP inflammatory pathways, their levels significantly change both in tissues and in the bloodstream during aging and are associated with a variety of inflammatory conditions. Decline in the expression of this miRNAs has been associated with aging and they have been proposed as biomarkers of aging related diseases.

The primers used for each of the genes were commercially available TaqMan Advanced miRNA Assay probes. The experiment was performed in vitro HEKa cells, one concentration of the peptide was tested (0.01 mg/mL) at 24 h.

Briefly, human epidermal keratinocytes from adult (HEKa) were seeded in 6-well plates at a density of 300,000 cells/well and maintained at standard culture conditions (DCBM medium supplemented with keratinocyte Growth Kit and 0.1% Penicillin/Streptomycin; 37°C, 95% Relative Humidity, 5% CO$_2$) for 24 hours. Next, HEKa were incubated in the presence of 0.01 mg/mL of the peptide for 24 h.

Cells were finally lysed and RNA extraction was performed using miRVana miRNA isolation kit following manufacturer's instructions. Purified miRNAs were used to generate the corresponding cDNAs by reverse transcription which served as templates for amplification. RT-qPCR was performed with the panel of TaqMan Advanced miRNA Assay probes specified above and Taqman Fast Advance Master Mix using StepOne plus Real-Time PCR instrument. Amplification included 40 cycles of: 1 second at 95°C (denaturation) and 20 seconds at 60°C (Annealing and extension).

The obtained data were analysed using the ΔΔCt method, which provides the target gene expression values as fold changes in the treated sample compared with an untreated basal sample. All samples were normalized with the relative expression of a housekeeping miRNA: miR-16-5p.

The steps for analysis included:

  1. Calculate the average Ct for each condition
  2. Calculate the ΔCT test sample and the ΔCT calibrator sample
  3. Calculate the ΔΔCT: ΔΔCT = ΔCT test sample - ΔCT calibrator sample
  4. Obtain ratio by $2^{-\Delta\Delta CT}$

Three independent experiments (N=3) were performed.

Obtained results appear summarized in Figure 3, which shows that the peptide SEQ ID NO: 2-NH$_2$ (as an example of the peptides of the present invention) was able to modulate the expression of miRNAs with anti-inflammatory properties. More precisely, the peptide of the present invention was able to upregulate the expression of both miR-146a-5p and miR-21-5p. All these results validate or demonstrate the usefulness of the peptides of the present invention against inflammation and, hence, for the prevention, reduction and/or treatment of inflammaging and skin aging. In addition, all these results also demonstrate the usefulness of the peptides of the present invention for the treatment of age-related diseases and inflammatory diseases, as explained above. Also, this anti-inflammatory activity demonstrates the usefulness of the peptides of the present invention as nutraceuticals

**Example 4. Analysis of the levels of IL-8**

For this example, peptides SEQ ID NO: 2-NH$_2$ and SEQ ID NO: 3-NH$_2$ were prepared in accordance with example 1.

A stock solution of the peptide was prepared in DMSO at 50 mg/mL. This stock was further diluted to obtain the final working concentrations of 0.005, 0.01 and 0.05 mg/mL in complete medium (DMEM supplemented with 1% FBS and 0.1% Penicillin/Streptomycin). Cells treated with 10 ng/mL IFNy alone were used as positive control for inflammation induction. Untreated cells were used as basal condition.

For this example, HaCaT keratinocytes were seeded in a 96-well plate at a density f 75,000 cells/well and maintained at standard culture conditions (DMEM with 10% FBS and 1% Penicillin/Streptomycin; 37°C, 95% RH, 5% CO$_2$) for 24 hours. Then keratinocytes were treated for 48 hours with the peptide of the present invention at the concentrations noted above in the presence of 10 ng/mL IFNy in DMEM +1% FBS. After the treatment, supernatants were collected and centrifuged in order to eliminate cell debris and were kept at -80°C until they were used for IL-8 quantification with Human IL-8/CXCL8 DuoSet ELISA kit according to the manufacturer instructions.

In order to determine cell viability, at the end of the experiment, cells were cultured in 0.5 mg/mL MTT in complete medium for 1 hour before removing MTT solution and dissolving formazan crystals formed with 150 μL DMSO/well. Finally, OD at 570 nm was determined with spectrophotometer Multiskan.

Each condition was performed in triplicate (n=3) in three independent experiments (N=3). All the obtained data was normalized in accordance with formulas (II) and (III):

$$IL - 8\,released\,per\,cell = \frac{IL - 8\,released\,per\,well}{OD570\,(MTT)}$$

$$(II)$$

$$\%\,vs.\,Basal = \frac{IL - 8\,released\,per\,cell\,in\,treated\,samples}{IL - 8\,released\,per\,cell\,in\,untreated\,samples\,(Basal)}\,X\,100$$

$$(III)$$

The obtained results appear summarized in Figures 4 and 5, which show that the peptides SEQ ID NO: 2-NH$_2$ and SEQ ID NO: 3-NH$_2$ (as an example of the peptides of the present invention) were able to decrease, in a dose-dependent manner, the secretion of IL-8 in all the conditions tested (treatment of 48 hours, at 0.005, 0.01 or 0.05 mg/mL together with IFNy at 10 ng/mL), compared to the positive control (inflammation induced with IFNy at 10 ng/mL).

A similar experiment was carried out under basal conditions (this is, without the inflammatory stimuli of IFNy), both for peptides SEQ ID NO: 2-NH$_2$ (concentrations of 0.005, 0.01 and 0.05 mg/mL) and SEQ ID NO: 3-NH$_2$ (concentrations of 0.01 and 0.05 mg/mL). In this case, the basal or untreated cells were stablished as 100% (IL-8 secreted by the basal or untreated cells). Obtained results appear summarized in Figures 6 and 7. As in the previous case, all the concentrations tested of SEQ ID NO: 2-NH$_2$ decreased the secretion of IL-8 in a dose-dependent manner but at a lower degree that under inflammatory conditions. In addition, and surprisingly, all concentrations tested of SEQ ID NO: 3-NH$_2$ also decreased the secretion of IL-8, but to a higher degree than SEQ ID NO: 2-NH$_2$.

All the results obtained in this example, demonstrate a protective effect of the peptides of the present invention as IL-8 is a proinflammatory cytokine and, hence, peptides of the present invention are useful for the prevention, reduction and/or treatment of inflammaging and skin aging. In addition, all these results also demonstrate the usefulness of the peptides of the present invention for the treatment of age-related diseases and inflammatory diseases, as explained above. Also, this anti-inflammatory activity demonstrates the usefulness of the peptides of the present invention as nutraceuticals

**Example 5. Analysis of the telomere length**

For this example, peptide SEQ ID NO: 2-NH$_2$ was prepared in accordance with example 1.

A stock solution of the peptide was prepared in DMSO at 50 mg/mL. This stock was further diluted to obtain the final working concentrations of 0.005 or 0.01 mg/mL in complete medium (Medium 106 supplemented with 2% LSGS). HDFa$_{73}$ (HDFa from 73 years old adult) untreated were used as negative control and HDFa$_{25}$ (HDFa from 25 years old adult untreated were considered for comparative purpose.

Briefly, Human Dermal fibroblasts from adult (HDFa) were seeded in 12-well plates at a concentration of 150,000 cells/well and maintained at standard culture conditions (Medium 106 supplemented with 2% LSGS, 37°C, 95% RH, 5% $CO_2$) for 24 hours.

Next, HDFa cells were treated with the above-mentioned peptide at the concentrations noted above, for 48 hours. Then, DNA was extracted using Qiagen DNeasy Mini kit following manufacturer's instructions. Finally, telomere DNA vs nuclear DNA ratio was stablished using Absolute Human Telomere Length Quantification qPCR Assay Kit, according to manufacturer instructions. The obtained data was analysed using the $\Delta\Delta Ct$ method, which provides the relative telomere length of the target sample as fold changes of telomere sequence (telDNA) compared with a single copy nuclear reference DNA sequence (SCR).

The steps for analysis included:

1. Calculate the Ct of each condition for telDNA and SCR
2. Calculate the $\Delta CT$ of telDNA (Ct telDNA target sample-Ct telDNA reference sample) and the $\Delta CT$ SCR DNA (Ct SCR target sample - Ct SCR reference sample)
3. Calculate the $\Delta\Delta CT$: $\Delta\Delta CT = \Delta CT$ tel DNA - $\Delta CT$ SCR DNA
4. Obtain relative telomere length by $2^{-\Delta\Delta CT}$
5. Calculate telomere length (%) = (relative telomere length of target sample / relative telomere length of $HDFa_{25}$ sample) $\times$ 100

Three independent experiments (N=3) were performed.

Obtained results appear summarized in Figure 8. $HDFa_{73}$ presented telomeres 20% shorted than $HDFa_{25}$. However, $HDFa_{73}$ incubated with 0.005 or 0.01 mg/mL of peptide SEQ ID NO: 2-$NH_2$ (as an example of the peptides of the present invention) for 48 hours showed telomeres a 17% and 30% longer than untreated $HDFa_{73}$, respectively. Therefore, the peptides of the present invention were able to induce telomere length recovery and restore telomere length of $HDFa_{73}$ to a similar level to that observed in $HDFa_{25}$, validating their usefulness for preventing, reducing and/or treating inflammaging and skin aging, as well as age-related diseases, inflammatory diseases and diseases related to oxidative stress.

**Example 6. Analysis of the subcellular localization of Nrf2**

For this example, peptide SEQ ID NO: 2-$NH_2$ was prepared in accordance with example 1.

A stock solution of the peptide was prepared in DMSO at 50 mg/mL. This stock was further diluted to obtain the final working concentrations of 0.01 or 0.05 mg/mL in complete medium (Dermal Basal Medium supplemented with keratinocyte Growth Kit and 0.1% Penicillin/Streptomycin). Untreated cells were used as negative control samples (basal condition).

For this study, HEKa cells were cultured in Dermal Cell Basal Medium (DCBM) at a density of 50,000 cell/well and incubated at 37°C in a humidified atmosphere with 5% $CO_2$. When cell confluence was around 80% cells were treated with the peptide at the different concentration noted above for 24 hours. After the treatment, samples were washed twice with PBS, fixed with 4% formaldehyde and permeabilized with Triton X-100 0.2%. Next, samples were blocked for 1 hour with PBS/BSA 5% in order to avoid nonspecific antibody binding.

For Nrf2 staining, samples were incubated overnight with 1:100 recombinant Alexa Fluor® 488 Anti-NRF2 antibody in PBS/BSA 1% at 4°C. After proper washing with PBS, cells were incubated with 50$\mu$g/mL Phalloidin-TRITC (for staining actin filaments) during 1 hour at room temperature and dark conditions. Finally, samples were washed three times with PBS and coverslips were mounted using Prolong-Gold with DAPI, which strongly binds DNA (nucleic staining), and samples were kept protected from light at 4°C until microscopic images were acquired. Microscopic images were acquired using 10x objective. Immunofluorescence was performed on thee replicates for each condition and images of at least five different fields from each coverslip were acquired using the same settings.

The obtained images were analized with Image J software for their fluorescence intensity in the nuclei (DAPI staining) and in the cytoplasm (Nrf2 staining).

The percentage of nuclear located Nrf2 was obtained by normalizing the nuclear fluorescence signal obtained for Nrf2 in the treated samples with regard to the one compared for the untreated sample.

Obtained results appear summarized in Figure 9, wherein it can be seen that peptide SEQ ID NO: 2-$NH_2$ (as an example of the peptides of the present invention) is able to provide for Nrf2 translocation from the cytoplasm to the nuclei in a concentration dependent manner. As it is widely known, in the nuclei, Nrf2 in the nuclei induces the expression of different antioxidant genes. Therefore, the peptides of the present invention are able to provide for anti-inflammatory and anti-oxidative activities which make them useful for the prevention, reductions and/or treatment of inflammaging and skin aging. In addition, all these results also demonstrate the usefulness of the peptides of the present invention for the treatment of age-related diseases, inflammatory diseases and diseases related to oxidative stress, as explained above. Also, this anti-inflammatory and antioxidant activities demonstrate the usefulness of the peptides of the present invention as nutraceuticals

### Example 7. Analysis of TLR2 levels in human epidermal keratinocytes

For this example, peptide SEQ ID NO: 2-NH$_2$ was prepared in accordance with example 1.

A stock solution of the peptide was prepared in DMSO at 50 mg/mL. This stock was further diluted to obtain the final working concentrations of 0.01 or 0.05 mg/mL in complete medium (Dermal Basal Medium supplemented with keratinocyte Growth Kit and 0.1% Penicillin/Streptomycin). Cells treated with 10 ng/mL IFNy alone were used as positive control samples for inflammation induction. Untreated cells were used as negative control samples (basal condition).

For this study, HEKa cells were seeded in a 24-well plate at a density of 50,000 cell/well and maintained at standard conditions (DCBM supplemented with keratinocytes growth kit and 0.1% Penicillin/Streptomycin; 37°C, 95% RH, 5% CO$_2$) for 24 hours. Then, keratinocytes were treated for 24 hours with 10 ng/mL IFNy. Next IFNy was removed and cells were incubated with different concentrations of the peptide of the present invention, as noted above, for 24 hours. Positive control samples (IFNy alone) were incubated with complete medium without peptide during this time. After the treatment, cells were washed with Dubelcco PBS (PBS also including potassium chloride) and lysed with RIPA and a scrapper. Lysates were collected and centrifuged in order to eliminate cell debris and protein concentrates were kept at -80°C until they were used for TLR2 quantification with Human TLR2 DuoSet ELISA kit according to manufacturer instructions.

Each condition was performed in triplicate (n=3) in three independent experiments (N=3).

Results appear summarized in Figure 10, wherein it can be seen that peptide SEQ ID NO: 2-NH$_2$ (as an example of the peptides of the present invention) is able to lower the levels of TLR2 after exposure to IFN$\gamma$ in a dose dependent-manner, showing up to 19% decrease after treatment with the highest peptide concentration. Therefore, the peptides of the present invention have a protective role against inflammation of the skin and are useful for the prevention, reduction and/or treatment of inflammaging and skin aging. In addition, all these results also demonstrate the usefulness of the peptides of the present invention for the treatment of age-related diseases and inflammatory diseases, as explained above. Also, this anti-inflammatory activity demonstrates the usefulness of the peptides of the present invention as nutraceuticals.

### Example 8. Analysis of the levels or synthesis of catalase enzyme

For this example, peptide SEQ ID NO: 2-NH$_2$ was prepared in accordance with example 1.

A stock solution of the peptide was prepared in DMSO at 50 mg/mL. This stock was further diluted to obtain the final working concentrations of 0.005, 0.01 or 0.05 mg/mL in complete medium (Dermal Basal Medium supplemented with keratinocyte Growth Kit and 0.1% Penicillin/Streptomycin). Untreated cells were used as negative control samples (basal condition).

For this study, HEKa cells were seeded in a 24-well plate at a density of 50,000 cell/well and maintained at standard culture conditions (DCBM; 37°C, 95% RH, 5% CO$_2$) for 24 hours. Then, keratinocytes were treated for 24 hours with the peptide of the present invention at the concentrations noted above. After treatment, cells were washed with Dubelcco PBS and lysed with RIPA and a scrapper. Lysates were collected and centrifuged in order to eliminate cell debris and protein concentrates were kept at -80°C until they were used for catalase quantification with human catalase ELISA Kit, according to manufacturer instructions. Finally, in order to normalize data, total protein content of each sample was quantified through Micro BCA Protein Assay kit, following manufacturer instructions.

Each condition was performed at least in triplicate (n=3) in three independent experiments (N=3).

Obtained results appear summarized in Figure 11, wherein it can be seen that peptide SEQ ID NO: 2-NH$_2$ (as an example of the peptides of the present invention) is able to increase the levels of catalase in a dose-dependent manner arriving at a 47% increase at the highest concentration. These results show the capacity of the peptides of the present invention provide an antioxidant response and, hence, demonstrates their usefulness for the prevention, reduction and/or treatment of inflammaging and skin aging. This antioxidant activity also demonstrates the usefulness of the peptides of the present invention for the treatment of diseases related to oxidative stress (as explained above) and as nutraceuticals.

### Example 9. Analysis of TRF1 levels

For this example, peptides SEQ ID NO: 2-NH$_2$ and SEQ ID NO: 3-NH$_2$ were prepared in accordance with example 1.

As it is widely known in the state of the art, in vertebrates, telomeres are composed by a tandem repeat of the TTAGGG sequence, bound by a six-protein complex known as shelterin which encompasses TRF1, TRF2, TIN2, POT1, RAP1 and TPP1. The Telomere Repeat Binding factor 1 (TRF1) was the first shelterin to be discovered (Zhong, Z., et al. (1992) A mammalian factor that binds telomeric TTAGGG repeats in vitro. Mol Cell Biol 12, 4834-4843.) and it is one of the best characterized. TRF1 binds to doublestranded TTAGGG repeats and its abundance at telomeres is proportional to the length of repeats (de Lange, T. (2005) Shelterin: the protein complex that shapes and safeguards human telomeres. Genes. Dev. 19, 2100-2110 and Liu, L., et al. (2007) Telomere lengthening early in development. Nat. Cell Biol. 9:1436-1441) being a good reporter of telomere length in vivo.

10,000 CHA9.3 cells were seeded per well in a sterile 96-well black-clear bottom tissue culture plate using 150 $\mu$L of DMEM medium supplemented with 10 % fetal bovine serum. Cells were incubated at 37°C, 5% $CO_2$/ humidified air for 24 h.

In parallel, peptides to be used in this example were weighed out and diluted in dimethyl sulfoxide (DMSO) or water to generate a "mother" plate. From here, an intermediate dilution plate was prepared. 1.5 $\mu$L of each compound solution was dispensed automatically using a Biomek FX Dual Pod automated liquid handling robot from the intermediated plate to the media of plated cells to get the final concentration 0.125 mg/mL for peptide SEQ ID NO: 2-$NH_2$ and 0.063 mg/mL for peptide SEQ ID NO: 3-$NH_2$. Each compound was assayed in duplicate. This final concentration for the screening was decided based on viability assays. Water or DMSO controls were also included in each plate as basal condition.

After a 24-hour incubation at 37°C, 5% $CO_2$ humidified air, cells were processed for immunofluorescence. Briefly, cells were fixed with 4% formaldehyde for 13 minutes in phosphate-buffered saline (PBS) and washed two times again with PBS at room temperature. Cells were next permeabilized with 0.2% of Triton X-100 detergent during 10 minutes, washed again with PBS and permeabilized with 0.1% sodium citrate plus 0.1 % of Triton X-100 for 5 minutes, the remaining buffer was removed and a final wash was performed. Cells were blocked with 50 $\mu$M of PBS-Tween-20 at 0.05% plus 3% of bovine serum albumin during 60 minutes at room temperature. Immunostaining was performed by incubation with anti-TRF1 primary antibody overnight at 4°C. After that, samples were washed 3 times with 0.05% PBS-Tween-20, and secondary antibody anti-rat-555 was dispensed at 1:500 in PBS-Tween plus 3% BSA during 60 minutes. Then, samples were washed 3 times with 0.05% PBS-Tween-20 plus another wash with PBS. Finally, an incubation with Hoechst staining 1 $\mu$g/mL to generate masks matching the cell nuclei was performed. SPSS software was used for statistical analysis. Image acquisition was performed on an Opera™ LX spinning disk confocal automated platform (PerkinElmer). Forty images were acquired with a 40x magnification lens under non-saturating conditions and an average of $1 \times 10^3$ cells were analyzed from each well. Quantification of fluorescence at single cell level was done with the Acapella™ platform using built-in analysis algorithms for the identification of foci and nuclei area and intensity.

Cell viability for each condition was also evaluated by comparing the number of nuclei with regards to controls.

Each condition was performed at least in duplicate (n=2) in three independent experiments (N=3). For all data mean TRF1 intensity values of the different experiments were calculated.

Obtained results appear summarized in Figures 12. As it can be directly derived from said Figure, the peptides of the present invention (as exemplified by peptides SEQ ID NO: 2-$NH_2$ and SEQ ID NO: 3-$NH_2$) provide for an increase in TRF1, which, as noted above, is directly related with telomere length (increase in telomere length), validating their usefulness of the peptides of the present invention for preventing, reducing and/or treating inflammaging and skin aging, as well as age-related diseases, inflammatory diseases and diseases related to oxidative stress.

**Example 10. Analysis of TRF1 and DNA damage under oxidative stress conditions**

For this example, peptide SEQ ID NO: 2-$NH_2$ was prepared in accordance with example 1.

A stock solution of the peptide prepared in DMSO at 50 mg/mL. This stock was further diluted to obtain the final working concentrations of 0.005 mg/mL, 0.01 mg/mL and 0.05 mg/mL of pure active.

HEKa cells were seeded on coverslips in 24-well plates containing Dermal Cell Basal Medium. Cultures were then kept in the 37°C incubator to attach and expand. When cell confluence was around 50%, media was replaced by media comprising the peptide at different working concentrations tested (5, 10 or 50 $\mu$g/mL of peptide SEQ ID NO: 2-$NH_2$) during 24h. Afterwards, media was replaced by a new media with the compound (for the wells in the basal condition experiment) or media with 100 $\mu$M $H_2O_2$ and the compound (for the wells in the oxidative stress condition), for another 24 h.

After treatment, cells were harvested, washed once with PBS, and treated for 30 seconds with Triton X-100 buffer for nuclear extraction and then washed again in PBS, fixed for 10 min in 4% buffered formaldehyde, washed in PBS and kept at 4°C until processed.

For immunofluorescence (IF), cells were permeabilized with 0.2% PBS-Triton for 10 min, washed and blocked with fetal bovine serum for one hour and 5% BSA in PBS for another 1h in order to avoid nonspecific antibody binding.

For TRF1 and 53BP1 staining, samples were incubated overnight at 4°C with the corresponding primary antibody. After proper washing with PBS, cells were incubated with 488-Alexa secondary for 1h at RT in a humid chamber. Samples in cover slides were mounted in Prolong Gold with DAPI which strongly binds DNA (nuclei staining), and samples were kept protected from light at 4°C until microscopic images were acquired. Fluorescent signals on slides were visualized in a confocal ultra-spectral microscope SP5-MP (Leica). Microscopic images were acquired using 40x objective. Immunofluorescence was performed on three replicates for each condition and images of at least four different fields from each coverslip were acquired using the same settings.

Signal was quantified using the Definiens Developer XD.2 Software. Image analysis was performed blindly. Blue channel (DAPI staining) was used to count the number of nuclei per image. Red and green colour were used to determine TRF1 and 53BP1 intensity, respectively. Then, number of TRF1 or 53BP1 foci was divided by the number of nuclei in order to obtain the total number of TRF1 or 53BP1 foci/nucleus of every image. Finally, all data was normalized with regard to the negative control: basal sample for TRF1 and sample treated with $H_2O_2$ for 53BP1, to obtain the % of TRF1 or 53BP1 foci per nucleus.

The results obtained for TRF1 appear summarized in Figure 13. As it can be readily ascertained from said figure, the peptides of the present invention (as exemplified by peptides SEQ ID NO: 2-NH$_2$) provide for an increase in TRF1, which, as noted above, is directly related with telomere length (increase in telomere length), validating their usefulness of the peptides of the present invention for preventing, reducing and/or treating inflammaging and skin aging.

The results obtained for 53BP1 appear summarized in Figure 14. As it can be readily ascertained from said figure, the peptides of the present invention (as exemplified by peptides SEQ ID NO: 2-NH$_2$

**Claims**

1. Peptide of formula (I):

$$R_1\text{-AA1-AA2-AA3-AA4-AA5-AA6-AA7-AA8-}R_2 \qquad (I)$$

its acceptable isomers, salts, solvates, derivatives and/or mixtures thereof, wherein

AA1 is a small amino acid;
AA2 is selected from polar uncharged or aromatic amino acids;
AA3 is absent or selected from aromatic amino acids;
AA4 is selected from negatively charged amino acids;
AA5 is selected from apolar amino acids;
AA6 is selected from Asn or Leu;
AA7 is selected from negatively charged amino acid;
AA8 is absent or selected from aromatic amino acids;
$R_1$ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and $R_5$-CO-, wherein $R_5$ is selected from the group formed by substituted or unsubstituted $C_1$-$C_{24}$ alkyl radical, substituted or unsubstituted $C_2$-$C_{24}$ alkenyl, substituted or unsubstituted $C_2$-$C_{24}$ alkynyl, substituted or unsubstituted $C_3$-$C_{24}$ cycloalkyl, substituted or unsubstituted $C_5$-$C_{24}$ cycloalkenyl, substituted or unsubstituted $C_8$-$C_{24}$ cycloalkynyl, substituted or unsubstituted $C_6$-$C_{30}$ aryl, substituted or unsubstituted $C_7$-$C_{24}$ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
$R_2$ is selected from H, -NR$_3$R$_4$- , -OR$_3$ and -SR$_3$, wherein $R_3$ and $R_4$ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl;

alternatively, $R_1$ and $R_2$ are absent and AA1 is directly bound to the last amino acid of the peptide, this is, AA8 or AA7 in case AA8 is absent (forming a cyclic peptide).

2. Peptide in accordance with claim 1, **characterized in that** in formula (I):

AA1 is selected from Cys, Val, Ala, Pro, Ser, Asn, Ala, Gly or Thr;
AA2 is selected from Tyr, Ser, Thr, Gln, Asn, Cys or Gly;
AA3 is absent or selected from Tyr, Phe or Trp;
AA4 is selected from Asp or Glu;
AA5 is selected from Ala, Val, Ile, Leu, Met, Pro, Phe or Trp;
AA6 is selected from Asn or Leu;

AA7 is selected from Asp or Glu;
AA8 is absent or selected from Tyr, Phe or Trp;

3. Peptide in accordance with claim 1 or claim 2, **characterized in that** in formula (I):

AA1 is selected from Ser, Cys, Ala or Gly;
AA2 is selected from Tyr, Trp, Ser, Gln or Asn;
AA3 is absent or selected from Tyr, Phe or Trp;
AA4 is selected from Asp or Glu;
AA5 is selected from Ala, Val, Ile or Leu;
AA6 is selected from Asn or Leu;
AA7 is selected from Asp or Glu;
AA8 is absent or selected from Tyr, Phe or Trp;

4. Peptide in accordance with any one of claims 1 to 3, **characterized in that** in formula (I):

AA1 is Gly;
AA2 is selected from Tyr or Asn;
AA3 is absent or Tyr;
AA4 is Glu;
AA5 is Leu;
AA6 is selected from Asn or Leu;
AA7 is Asp;
AA8 is absent or Tyr.

5. Peptide in accordance with any one of claims 1 to 4, **characterized in that** the peptide is selected from:

$R_1$-Gly-Tyr-Tyr-Glu-Leu-Asn-Asp-$R_2$ ($R_1$-SEQ ID NO: 2-$R_2$); or
$R_1$-Gly-Asn-Glu-Leu-Leu-Asp-Tyr-$R_2$ ($R_1$-SEQ ID NO: 3-$R_2$)

6. Peptide in accordance with any one of claims 1 to 5, **characterized in that** $R_1$ is H.

7. Peptide in accordance with any one of claims 1 to 6, **characterized in that** $R_2$ is $NH_2$.

8. Composition comprising a peptide in accordance with any one of claims 1 to 7.

9. Use as a cosmetic of a peptide in accordance with any one of claims 1 to 7 or of a composition in accordance with claim 8 in a subject in need thereof.

10. Use as a cosmetic in accordance with claim 9, **characterized in that** the use as a cosmetic is: to prevent, reduce and/or treat skin aging; and/or to prevent, reduce and/or treat inflammaging; and/or for the improvement of skin firmness and/or elasticity.

11. Use a cosmetic in accordance with claim 10, **characterized in that** the signs of skin aging are wrinkles, skin roughness, looser skin or sagging skin.

12. Composition in accordance with claim 8 or peptide in accordance with any one of claims 1 to 7 for use as a medicament.

13. Composition in accordance with claim 8 or peptide in accordance with any one of claims 1 to 7 for use in the prevention, amelioration and/or treatment of an age-related disease, an inflammatory disease or a disease related to oxidative-stress

14. Use as a nutraceutical of a peptide in accordance with any one of claims 1 to 7 or of a composition in accordance with claim 8.

15. Nutraceutical comprising a peptide in accordance with any one of claims 1 to 7.

Fig. 1

Fig. 2

Fig. 3

Fig. 4

Fig. 5

Fig. 6

*Fig. 7*

*Fig. 8*

Fig. 9

Fig. 10

Fig. 11

Fig. 12

Fig. 13

Fig. 14

**EP 4 249 498 A1**

## EUROPEAN SEARCH REPORT

**Application Number**

EP 22 38 2267

Europäisches Patentamt
European Patent Office
Office européen des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/040152 A1 (LIU JUN-PING [CN]) 11 February 2021 (2021-02-11) | 1-4,6-15 | INV. C07K7/06 |
| A | * paragraphs [0007] - [0011], [0014] - [0016]; claims 1-11; examples 1,4; table 1 * | 5 | A23L33/18 A61K8/64 A61P17/00 |
| X | WO 2007/101227 A2 (UNIV ARIZONA STATE [US]; JOHNSTON STEPHEN A [US]; LAKE DOUG [US]) 7 September 2007 (2007-09-07) * page 75; sequence 36 * | 1,2,6,8, 12,13,15 | ADD. A61K38/00 |
| X | WO 03/099196 A2 (CURE TECH LTD [IL]; MOR RESEARCH APPLIC LTD [IL] ET AL.) 4 December 2003 (2003-12-04) * sequence 14 * | 1,2,6,8, 15 | |
| X | KR 2014 0089295 A (KAEL GEMVAX CO LTD [KR]; KIM SANG JAE [KR]) 14 July 2014 (2014-07-14) | 1 | |
| A | * paragraphs [0004] - [0027], [0059] - [0081]; claims 1-8 * | 2-15 | TECHNICAL FIELDS SEARCHED (IPC) |
| X | KR 2018 0027307 A (TAEIL [KR]) 14 March 2018 (2018-03-14) | 1 | C07K A61K |
| A | * paragraphs [0014], [0033] - [0046]; claims 1-5; example 1 * | 2-15 | A23L A61Q A61P |
| X | WO 2019/166347 A1 (LIPOTRUE S L [ES]) 6 September 2019 (2019-09-06) | 1 | |
| A | * page 4 - page 5; claims 1-14,18 * | 2-15 | |
| X | US 2003/113388 A1 (PHAN DUNG [US]) 19 June 2003 (2003-06-19) | 1 | |
| A | * paragraphs [0006], [0092]; claims 1-30 * | 2-15 | |

-/--

The present search report has been drawn up for all claims

| Place of search | Date of completion of the search | Examiner |
|---|---|---|
| The Hague | 9 August 2022 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

31

Europäisches
Patentamt

European
Patent Office

Office européen
des brevets

**EUROPEAN SEARCH REPORT**

Application Number

EP 22 38 2267

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "UPI001C718825 ¦ UniParc ¦ UniProt", , 18 August 2021 (2021-08-18), XP055949757, Retrieved from the Internet: URL:https://www.uniprot.org/uniparc/UPI001C718825/entry [retrieved on 2022-08-08] | 1 | |
| A | * abstract * | 2-15 | |
| X | Anonymous: "UPI000B148A05 ¦ UniParc ¦ UniProt", , 6 April 2016 (2016-04-06), XP055949760, Retrieved from the Internet: URL:https://www.uniprot.org/uniparc/UPI000B148A05/entry [retrieved on 2022-08-08] | 1 | |
| A | * abstract * | 2-15 | |

TECHNICAL FIELDS
SEARCHED     (IPC)

The present search report has been drawn up for all claims

| Place of search | Date of completion of the search | Examiner |
|---|---|---|
| The Hague | 9 August 2022 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**EP 4 249 498 A1**

## ANNEX TO THE EUROPEAN SEARCH REPORT
## ON EUROPEAN PATENT APPLICATION NO.

EP 22 38 2267

09-08-2022

| Patent document cited in search report | | | Publication date | Patent family member(s) | | | Publication date |
|---|---|---|---|---|---|---|---|
| US 2021040152 | A1 | | 11-02-2021 | CN | 108129553 | A | 08-06-2018 |
| | | | | US | 2021040152 | A1 | 11-02-2021 |
| | | | | WO | 2019134574 | A1 | 11-07-2019 |
| WO 2007101227 | A2 | | 07-09-2007 | AU | 2007220042 | A1 | 07-09-2007 |
| | | | | CA | 2651796 | A1 | 07-09-2007 |
| | | | | EP | 1994181 | A2 | 26-11-2008 |
| | | | | JP | 2009532664 | A | 10-09-2009 |
| | | | | US | 2009186042 | A1 | 23-07-2009 |
| | | | | US | 2013072660 | A1 | 21-03-2013 |
| | | | | WO | 2007101227 | A2 | 07-09-2007 |
| WO 03099196 | A2 | | 04-12-2003 | AU | 2003230183 | A1 | 12-12-2003 |
| | | | | CA | 2487060 | A1 | 04-12-2003 |
| | | | | CY | 1117114 | T1 | 05-04-2017 |
| | | | | DK | 1575484 | T3 | 12-10-2015 |
| | | | | EP | 1575484 | A2 | 21-09-2005 |
| | | | | ES | 2549303 | T3 | 26-10-2015 |
| | | | | HU | E025710 | T2 | 28-04-2016 |
| | | | | IL | 165193 | A | 30-12-2010 |
| | | | | IL | 207985 | A | 31-10-2013 |
| | | | | JP | 4764627 | B2 | 07-09-2011 |
| | | | | JP | 2006505244 | A | 16-02-2006 |
| | | | | JP | 2011139708 | A | 21-07-2011 |
| | | | | KR | 20050008736 | A | 21-01-2005 |
| | | | | PT | 1575484 | E | 02-11-2015 |
| | | | | SI | 1575484 | T1 | 30-11-2015 |
| | | | | US | 2005180969 | A1 | 18-08-2005 |
| | | | | US | 2008025980 | A1 | 31-01-2008 |
| | | | | US | 2009263386 | A1 | 22-10-2009 |
| | | | | WO | 03099196 | A2 | 04-12-2003 |
| KR 20140089295 | A | | 14-07-2014 | NONE | | | |
| KR 20180027307 | A | | 14-03-2018 | NONE | | | |
| WO 2019166347 | A1 | | 06-09-2019 | AU | 2019228281 | A1 | 13-08-2020 |
| | | | | CA | 3090060 | A1 | 06-09-2019 |
| | | | | CN | 112040969 | A | 04-12-2020 |
| | | | | EP | 3758730 | A1 | 06-01-2021 |
| | | | | JP | 2021514940 | A | 17-06-2021 |
| | | | | KR | 20200124218 | A | 02-11-2020 |
| | | | | TW | 201941783 | A | 01-11-2019 |
| | | | | US | 2021032287 | A1 | 04-02-2021 |
| | | | | WO | 2019166347 | A1 | 06-09-2019 |

EPO FORM P0459

page 1 of 2

## ANNEX TO THE EUROPEAN SEARCH REPORT
## ON EUROPEAN PATENT APPLICATION NO.

EP 22 38 2267

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2022

| Patent document cited in search report | Publication date | Patent family member(s) | Publication date |
|---|---|---|---|
| US 2003113388 A1 | 19-06-2003 | NONE | |

For more details about this annex : see Official Journal of the European Patent Office, No. 12/82

EPO FORM P0459

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NGUYEN AV ; SOULIKA AM.** The Dynamics of the Skin's Immune System. *International journal of molecular sciences,* 2019, vol. 20 (8), 1811
- **LEE HS et al.** Anti-inflammatory effects of ethanol extract from the leaves and shoots of Cedrela odorata L. in cytokine-stimulated keratinocytes. *Experimental and therapeutic medicine,* 2019, vol. 18 (1), 833-840
- **GANCEVICIENE, R. et al.** Skin Anti-Aging Strategies. *Dermato-Endocrinology,* 2012, vol. 4, 308-319
- **ZHUANG Y ; LYGA J.** Inflammaging in skin and other tissues - the roles of complement system and macrophage. *Inflamm Allergy Drug Targets,* 2014, vol. 13, 153-61
- **XIA S et al.** An Update on Inflamm-Aging: Mechanisms, Prevention, and Treatment. *J Immunol Res.,* 2016, vol. 2016, 8426874
- **RÜBE, C.E. et al.** Human skin aging is associated with increased expression of the histone variant H2A.J in the epidermis. *Aging Mech Dis,* 2021, vol. 7, 7
- **FAGAGNA et al.** A DNA damage checkpoint response in telomere-initiated senescence. *Nature,* 2003, vol. 426, 194-198
- **DE MAGALHÃES J P ; PASSOS J F.** Stress, cell senescence and organismal ageing. *Mechanisms of Ageing and Development,* 2018, vol. 170, 2-9
- **KEMÉNY L et al.** Role of interleukin-8 receptor in skin. *International archives of allergy and immunology,* 1994, vol. 104 (4), 317-322
- **CHEN L ; DIPIETRO LA.** Toll-Like Receptor Function in Acute Wounds. *Adv Wound Care (New Rochelle),* 2017, vol. 6, 344-355
- **PIVARCSI A et al.** Expression and function of Toll-like receptors 2 and 4 in human keratinocytes. *Int Immunol.,* 2003, vol. 15, 721-30
- **BAILEY KL et al.** Aging leads to dysfunctional innate immune responses to TLR2 and TLR4 agonists. *Aging Clin Exp Res.,* 2019, vol. 31, 1185-1193
- **ZHANG B et al.** Toll-like receptor 2 plays a critical role in pathogenesis of acne vulgaris. *Biomed Dermatol,* 2019, vol. 3, 4
- **BUCKINGHAM EM ; KLINGELHUTZ AJ.** The role of telomeres in the ageing of human skin. *Exp Dermatol.,* 2011, vol. 20, 297-302
- **JIANG H et al.** Telomere shortening and ageing. *Z Gerontol Geriatr.,* 2007, vol. 40, 314-24

- **IMBERT I et al.** Modulation of telomere binding proteins: a future area of research for skin protection and anti-aging target. *J Cosmet Dermatol.,* 2012, vol. 11, 162-6
- **MALEKI M et al.** Stabilization of telomere by the antioxidant property of polyphenols: Anti-aging potential. *Life Sci.,* 2020, vol. 259, 118341
- **BLACKBUM, E. H.** Structure and function of telomeres. *Nature,* 1991, vol. 350, 569-573
- **LIU, L. et al.** Telomere lengthening early in development. *Nat. Cell Biol.,* 2007, vol. 9, 1436-1441
- **FLORES, I. et al.** Effects of telomerase and telomere length on epidermal stem cell behavior. *Science,* 2005, vol. 309, 1253-1256
- **MARION, R.M. et al.** Telomeres acquire embryonic stem cell characteristics in induced pluripotent stem cells. *Cell Stem Cell,* 2009, vol. 4, 141-54
- **LÓPEZ-OTÍN, C. et al.** The hallmarks of aging. *Cell,* 2013, vol. 153, 1194-1217
- **MARTINEZ, P ; BLASCO, M.A.** Telomere-driven diseases and telomere-targeting therapies. *J Cell Biol.,* 2017, vol. 216 (4), 875-887
- **SHARPLESS., N.E. ; DEPINHO, R.A.** How stem cells age and why this makes us grow old. *Nat. Rev. Mol. Cell Biol.,* 2007, vol. 8, 703-713
- **BRADLEY E.J. et al.** Over-the-counter anti-ageing topical agents and their ability to protect and repair photoaged skin. *Maturitas,* 2015, vol. 80, 265-272
- **MANELA-AZULAY M. et al.** Vitamins and other Antioxidants. *Daily Routine in Cosmetic Dermatology,* 2017, 1-13
- **ZHANG L. ; FALLA T.J.** Cosmeceuticals and peptides. *Clinics in dermatology,* 2009, vol. 27, 485-494
- Unusual amino acids in peptide synthesis. **D. C. ROBERTS ; F. VELLACCIO.** The Peptides. Academic Press, 1983, vol. 5
- *Eur. J. Biochem.,* 1984, vol. 138, 937
- **LLOYD WILLIAMS P. et al.** Chemical Approaches to the Synthesis of Peptides and Proteins. CRC, 1997
- **KAISER E. et al.** *Anal. Biochem.,* 1970, vol. 34, 595598
- **ZHONG, Z. et al.** A mammalian factor that binds telomeric TTAGGG repeats in vitro. *Mol Cell Biol,* 1992, vol. 12, 4834-4843

- **DE LANGE, T.** Shelterin: the protein complex that shapes and safeguards human telomeres. *Genes. Dev.,* 2005, vol. 19, 2100-2110